# EUROPEAN PATENT SPECIFICATION

(11) **EP 3 428 648 B1**
(45) Date of publication and mention of the grant of the patent: **02.12.2020**
(21) Application number: 17763179.3
(22) Date of filing: 06.03.2017
(51) Int. Cl.: G01N 33/574, C12M 1/34, G06Q 50/22, C07K 14/47, C07K 16/30

(54) **METHOD FOR ASSISTING PREDICTION OF RECURRENCE RISK IN HEPATOCELLULAR CARCINOMA PATIENT, AND USE OF A KIT**
VERFAHREN ZUR UNTERSTÜTZUNG DER VORHERSAGE DES REZIDIVRISIKOS BEI EINEM PATIENTEN MIT HEPATOZELLULÄREM KARZINOM, UND VERWENDUNG EINES KITS
PROCÉDÉ D'AIDE À LA PRÉDICTION D'UN RISQUE DE RÉCIDIVE CHEZ UN PATIENT ATTEINT D'UN CARCINOME HÉPATOCELLULAIRE, ET UTILISATION D'UN KIT

(30) Priority: 10.03.2016 JP 2016047026; 23.09.2016 JP 2016185635
(43) Date of publication of application: 16.01.2019
(73) Proprietor: National Cancer Center, Tokyo 104-0045 (JP); Sysmex Corporation, Kobe-shi, Hyogo 651-0073 (JP)
(72) Inventor: NAKATSURA, Tetsuya, Kashiwa-shi Chiba 277-8577 (JP); SAITO, Keigo, Kashiwa-shi Chiba 277-8577 (JP); MIURA, Masahiro, Kobe-shi Hyogo 651-0073 (JP); SUTO, Kozo, Kobe-shi Hyogo 651-0073 (JP); IINO, Takuya, Kobe-shi Hyogo 6510073 (JP)
(74) Representative: De Clercq & Partners
(86) International application number: PCT/JP2017/008799
(87) International publication number: WO 2017/154839

(56) References cited:
- WO-A1-2006/038588
- JP-A- 2005 526 979
- JP-A- 2015 526 387
- JP-A- 2016 095 233
- US-A1- 2006 014 223
- US-A1- 2009 060 907
- US-A1- 2015 368 340
- Kazuya Ofuji ET AL: "Pre-operative plasma glypican-3 levels detected by a novel ELISA system predict the risk of post-operative recurrence in patients with stage I hepatocellular carcinoma", Hepatology; vol. 62, 1 October 2015 (2015-10-01), page 464A, XP55621922, Retrieved from the Internet: URL:http://onlinelibrary.wiley.com/journal /10.1002/(ISSN)1527-3350 [retrieved on 2019-09-13]
- KAZUYA OFUJI ET AL: "Perioperative plasma glypican-3 level may enable prediction of the risk of recurrence after surgery in patients with stage I hepatocellular carcinoma", ONCOTARGET, vol. 8, no. 23, 6 June 2017 (2017-06-06), pages 37835-37844, XP55621951, DOI: 10.18632/oncotarget.14271
- KAZUYA OFUJI et al.: "Glypican-3 is useful for predicting the risk of post-operative recurrence in patients with stage I hepatocellular carcinoma", Journal of Japan Society for Molecular Tumor Marker Research, vol. 31, 25 December 2015 (2015-12-25), pages 30-31, XP055576603, DOI: 10.11241/jsmtmr.31.30

### SEQUENCE LISTING

<110> SYSMEX CORPORATION National Cancer Center
<120> Method for assisting the prediction of risk of recurrence of hepatocellular carcinoma
<130> 1-2015-071PCT
<150> JP2016-047026
   <151> 2016-03-10
<150> JP2016-185635
   <151> 2016-09-23
<160> 57
<170> PatentIn version 3.5
<210> 1
   <211> 20
   <212> PRT
   <213> Artificial Sequence
<220>
   <223> a.a. 1-20
<400> 1
   Met Ala Gly Thr Val Arg Thr Ala Cys Leu Val Val Ala Met Leu Leu
   1               5                   10                  15
   Ser Leu Asp Phe
               20
<210> 2
   <211> 20
   <212> PRT
   <213> Artificial Sequence
<220>
   <223> a.a.11-30
<400> 2
   Val Val Ala Met Leu Leu Ser Leu Asp Phe Pro Gly Gln Ala Gln Pro
   1               5                   10                  15
   Pro Pro Pro Pro
               20
<210> 3
   <211> 20
   <212> PRT
   <213> Artificial Sequence
<220>
   <223> aa25-44
<400> 3
   Gln Pro Pro Pro Pro Pro Pro Asp Ala Thr Cys His Gln Val Arg Ser
   1               5                   10                  15
   Phe Phe Gln Arg
               20
<210> 4
   <211> 20
   <212> PRT
   <213> Artificial Sequence
<220>
   <223> aa35-54
<400> 4
   Cys His Gln Val Arg Ser Phe Phe Gln Arg Leu Gln Pro Gly Leu Lys
   1               5                   10                  15
   Trp Val Pro Glu
               20
<210> 5
   <211> 20
   <212> PRT
   <213> Artificial Sequence
<220>
   <223> aa45-64
<400> 5
   Leu Gln Pro Gly Leu Lys Trp Val Pro Glu Thr Pro Val Pro Gly Ser
   1               5                   10                  15
   Asp Leu Gln Val
               20
<210> 6
   <211> 20
   <212> PRT
   <213> Artificial Sequence
<220>
   <223> aa55-74
<400> 6
   Thr Pro Val Pro Gly Ser Asp Leu Gln Val Cys Leu Pro Lys Gly Pro
   1               5                   10                  15
   Thr Cys Cys Ser
               20
<210> 7
   <211> 20
   <212> PRT
   <213> Artificial Sequence
<220>
   <223> aa65-84
<400> 7
   Cys Leu Pro Lys Gly Pro Thr Cys Cys Ser Arg Lys Met Glu Glu Lys
   1               5                   10                  15
   Tyr Gln Leu Thr
               20
<210> 8
   <211> 20
   <212> PRT
   <213> Artificial Sequence
<220>
   <223> aa75-94
<400> 8
   Arg Lys Met Glu Glu Lys Tyr Gln Leu Thr Ala Arg Leu Asn Met Glu
   1               5                   10                  15
   Gln Leu Leu Gln
               20
<210> 9
   <211> 20
   <212> PRT
   <213> Artificial Sequence
<220>
   <223> aa85-104
<400> 9
   Ala Arg Leu Asn Met Glu Gln Leu Leu Gln Ser Ala Ser Met Glu Leu
   1               5                   10                  15
   Lys Phe Leu Ile
               20
<210> 10
   <211> 20
   <212> PRT
   <213> Artificial Sequence
<220>
   <223> aa95-114
<400> 10
   Ser Ala Ser Met Glu Leu Lys Phe Leu Ile Ile Gln Asn Ala Ala Val
   1               5                   10                  15
   Phe Gln Glu Ala
               20
<210> 11
   <211> 20
   <212> PRT
   <213> Artificial Sequence
<220>
   <223> aa105-124
<400> 11
   Ile Gln Asn Ala Ala Val Phe Gln Glu Ala Phe Glu Ile Val Val Arg
   1               5                   10                  15
   His Ala Lys Asn
               20
<210> 12
   <211> 20
   <212> PRT
   <213> Artificial Sequence
<220>
   <223> aa115-134
<400> 12
   Phe Glu Ile Val Val Arg His Ala Lys Asn Tyr Thr Asn Ala Met Phe
   1               5                   10                  15
   Lys Asn Asn Tyr
               20
<210> 13
   <211> 20
   <212> PRT
   <213> Artificial Sequence
<220>
   <223> aa125-144
<400> 13
   Tyr Thr Asn Ala Met Phe Lys Asn Asn Tyr Pro Ser Leu Thr Pro Gln
   1               5                   10                  15
   Ala Phe Glu Phe
               20
<210> 14
   <211> 20
   <212> PRT
   <213> Artificial Sequence
<220>
   <223> aa135-154
<400> 14
   Pro Ser Leu Thr Pro Gln Ala Phe Glu Phe Val Gly Glu Phe Phe Thr
   1               5                   10                  15
   Asp Val Ser Leu
               20
<210> 15
   <211> 20
   <212> PRT
   <213> Artificial Sequence
<220>
   <223> aa145-164
<400> 15
   Val Gly Glu Phe Phe Thr Asp Val Ser Leu Tyr Ile Leu Gly Ser Asp
   1               5                   10                  15
   Ile Asn Val Asp
               20
<210> 16
   <211> 20
   <212> PRT
   <213> Artificial Sequence
<220>
   <223> aa155-174
<400> 16
   Tyr Ile Leu Gly Ser Asp Ile Asn Val Asp Asp Met Val Asn Glu Leu
   1               5                   10                  15
   Phe Asp Ser Leu
               20
<210> 17
   <211> 20
   <212> PRT
   <213> Artificial Sequence
<220>
   <223> aa165-184
<400> 17
   Asp Met Val Asn Glu Leu Phe Asp Ser Leu Phe Pro Val Ile Tyr Thr
   1               5                   10                  15
   Gln Leu Met Asn
               20
<210> 18
   <211> 20
   <212> PRT
   <213> Artificial Sequence
<220>
   <223> aa175-194
<400> 18
   Phe Pro Val Ile Tyr Thr Gln Leu Met Asn Pro Gly Leu Pro Asp Ser
   1               5                   10                  15
   Ala Leu Asp Ile
               20
<210> 19
   <211> 20
   <212> PRT
   <213> Artificial Sequence
<220>
   <223> aa185-204
<400> 19
   Pro Gly Leu Pro Asp Ser Ala Leu Asp Ile Asn Glu Cys Leu Arg Gly
   1 5 10 15
   Ala Arg Arg Asp
   20
<210> 20
   <211> 20
   <212> PRT
   <213> Artificial Sequence
<220>
   <223> aa195-214
<400> 20
   Asn Glu Cys Leu Arg Gly Ala Arg Arg Asp Leu Lys Val Phe Gly Asn
   1 5 10 15
   Phe Pro Lys Leu
   20
<210> 21
   <211> 20
   <212> PRT
   <213> Artificial Sequence
<220>
   <223> aa205-224
<400> 21
   Leu Lys Val Phe Gly Asn Phe Pro Lys Leu Ile Met Thr Gln Val Ser
   1 5 10 15
   Lys Ser Leu Gln
   20
<210> 22
   <211> 20
   <212> PRT
   <213> Artificial Sequence
<220>
   <223> aa215-234
<400> 22
   Ile Met Thr Gln Val Ser Lys Ser Leu Gln Val Thr Arg Ile Phe Leu
   1 5 10 15
   Gln Ala Leu Asn
   20
<210> 23
   <211> 20
   <212> PRT
   <213> Artificial Sequence
<220>
   <223> aa225-244
<400> 23
   Val Thr Arg Ile Phe Leu Gln Ala Leu Asn Leu Gly Ile Glu Val Ile
   1               5                   10                  15
   Asn Thr Thr Asp
               20
<210> 24
   <211> 20
   <212> PRT
   <213> Artificial Sequence
<220>
   <223> aa235-254
<400> 24
   Leu Gly Ile Glu Val Ile Asn Thr Thr Asp His Leu Lys Phe Ser Lys
   1               5                   10                  15
   Asp Cys Gly Arg
               20
<210> 25
   <211> 20
   <212> PRT
   <213> Artificial Sequence
<220>
   <223> aa245-264
<400> 25
   His Leu Lys Phe Ser Lys Asp Cys Gly Arg Met Leu Thr Arg Met Trp
   1               5                   10                  15
   Tyr Cys Ser Tyr
               20
<210> 26
   <211> 20
   <212> PRT
   <213> Artificial Sequence
<220>
   <223> aa255-274
<400> 26
   Met Leu Thr Arg Met Trp Tyr Cys Ser Tyr Cys Gln Gly Leu Met Met
   1               5                   10                  15
   Val Lys Pro Cys
               20
<210> 27
   <211> 20
   <212> PRT
   <213> Artificial Sequence
<220>
   <223> aa265-284
<400> 27
   Cys Gln Gly Leu Met Met Val Lys Pro Cys Gly Gly Tyr Cys Asn Val
   1               5                   10                  15
   Val Met Gln Gly
               20
<210> 28
   <211> 20
   <212> PRT
   <213> Artificial Sequence
<220>
   <223> aa275-294
<400> 28
   Gly Gly Tyr Cys Asn Val Val Met Gln Gly Cys Met Ala Gly Val Val
   1               5                   10                  15
   Glu Ile Asp Lys
               20
<210> 29
   <211> 20
   <212> PRT
   <213> Artificial Sequence
<220>
   <223> aa285-304
<400> 29
   Cys Met Ala Gly Val Val Glu Ile Asp Lys Tyr Trp Arg Glu Tyr Ile
   1               5                   10                  15
   Leu Ser Leu Glu
               20
<210> 30
   <211> 20
   <212> PRT
   <213> Artificial Sequence
<220>
   <223> aa295-314
<400> 30
   Tyr Trp Arg Glu Tyr Ile Leu Ser Leu Glu Glu Leu Val Asn Gly Met
   1               5                   10                  15
   Tyr Arg Ile Tyr
               20
<210> 31
   <211> 20
   <212> PRT
   <213> Artificial Sequence
<220>
   <223> aa305-324
<400> 31
   Glu Leu Val Asn Gly Met Tyr Arg Ile Tyr Asp Met Glu Asn Val Leu
   1               5                   10                  15
   Leu Gly Leu Phe
               20
<210> 32
   <211> 20
   <212> PRT
   <213> Artificial Sequence
<220>
   <223> aa315-334
<400> 32
   Asp Met Glu Asn Val Leu Leu Gly Leu Phe Ser Thr Ile His Asp Ser
   1               5                   10                  15
   Ile Gln Tyr Val
               20
<210> 33
   <211> 20
   <212> PRT
   <213> Artificial Sequence
<220>
   <223> aa325-344
<400> 33
   Ser Thr Ile His Asp Ser Ile Gln Tyr Val Gln Lys Asn Ala Gly Lys
   1               5                   10                  15
   Leu Thr Thr Thr
               20
<210> 34
   <211> 20
   <212> PRT
   <213> Artificial Sequence
<220>
   <223> aa335-354
<400> 34
   Gln Lys Asn Ala Gly Lys Leu Thr Thr Thr Ile Gly Lys Leu Cys Ala
   1               5                   10                  15
   His Ser Gln Gln
               20
<210> 35
   <211> 20
   <212> PRT
   <213> Artificial Sequence
<220>
   <223> aa345-364
<400> 35
   Ile Gly Lys Leu Cys Ala His Ser Gln Gln Arg Gln Tyr Arg Ser Ala
   1               5                   10                  15
   Tyr Tyr Pro Glu
               20
<210> 36
   <211> 20
   <212> PRT
   <213> Artificial Sequence
<220>
   <223> aa359-378
<400> 36
   Ser Ala Tyr Tyr Pro Glu Asp Leu Phe Ile Asp Lys Lys Val Leu Lys
   1 5 10 15
   Val Ala His Val
   20
<210> 37
   <211> 20
   <212> PRT
   <213> Artificial Sequence
<220>
   <223> aa369-388
<400> 37
   Asp Lys Lys Val Leu Lys Val Ala His Val Glu His Glu Glu Thr Leu
   1 5 10 15
   Ser Ser Arg Arg
   20
<210> 38
   <211> 20
   <212> PRT
   <213> Artificial Sequence
<220>
   <223> aa379-398
<400> 38
   Glu His Glu Glu Thr Leu Ser Ser Arg Arg Arg Glu Leu Ile Gln Lys
   1 5 10 15
   Leu Lys Ser Phe
   20
<210> 39
   <211> 20
   <212> PRT
   <213> Artificial Sequence
<220>
   <223> aa389-408
<400> 39
   Arg Glu Leu Ile Gln Lys Leu Lys Ser Phe Ile Ser Phe Tyr Ser Ala
   1 5 10 15
   Leu Pro Gly Tyr
   20
<210> 40
   <211> 20
   <212> PRT
   <213> Artificial Sequence
<220>
   <223> aa399-418
<400> 40
   Ile Ser Phe Tyr Ser Ala Leu Pro Gly Tyr Ile Cys Ser His Ser Pro
   1 5 10 15
   Val Ala Glu Asn
   20
<210> 41
   <211> 20
   <212> PRT
   <213> Artificial Sequence
<220>
   <223> aa409-428
<400> 41
   Ile Cys Ser His Ser Pro Val Ala Glu Asn Asp Thr Leu Cys Trp Asn
   1 5 10 15
   Gly Gln Glu Leu
   20
<210> 42
   <211> 20
   <212> PRT
   <213> Artificial Sequence
<220>
   <223> aa419-438
<400> 42
   Asp Thr Leu Cys Trp Asn Gly Gln Glu Leu Val Glu Arg Tyr Ser Gln
   1 5 10 15
   Lys Ala Ala Arg
   20
<210> 43
   <211> 20
   <212> PRT
   <213> Artificial Sequence
<220>
   <223> aa429-448
<400> 43
   Val Glu Arg Tyr Ser Gln Lys Ala Ala Arg Asn Gly Met Lys Asn Gln
   1               5                   10                  15
   Phe Asn Leu His
               20
<210> 44
   <211> 20
   <212> PRT
   <213> Artificial Sequence
<220>
   <223> aa439-458
<400> 44
   Asn Gly Met Lys Asn Gln Phe Asn Leu His Glu Leu Lys Met Lys Gly
   1               5                   10                  15
   Pro Glu Pro Val
               20
<210> 45
   <211> 20
   <212> PRT
   <213> Artificial Sequence
<220>
   <223> aa449-468
<400> 45
   Glu Leu Lys Met Lys Gly Pro Glu Pro Val Val Ser Gln Ile Ile Asp
   1               5                   10                  15
   Lys Leu Lys His
               20
<210> 46
   <211> 20
   <212> PRT
   <213> Artificial Sequence
<220>
   <223> aa459-478
<400> 46
   Val Ser Gln Ile Ile Asp Lys Leu Lys His Ile Asn Gln Leu Leu Arg
   1               5                   10                  15
   Thr Met Ser Met
               20
<210> 47
   <211> 20
   <212> PRT
   <213> Artificial Sequence
<220>
   <223> aa469-488
<400> 47
   Ile Asn Gln Leu Leu Arg Thr Met Ser Met Pro Lys Gly Arg Val Leu
   1               5                   10                  15
   Asp Lys Asn Leu
               20
<210> 48
   <211> 20
   <212> PRT
   <213> Artificial Sequence
<220>
   <223> aa483-502
<400> 48
   Val Leu Asp Lys Asn Leu Asp Glu Glu Gly Phe Glu Ser Gly Asp Cys
   1               5                   10                  15
   Gly Asp Asp Glu
               20
<210> 49
   <211> 20
   <212> PRT
   <213> Artificial Sequence
<220>
   <223> aa493-512
<400> 49
   Phe Glu Ser Gly Asp Cys Gly Asp Asp Glu Asp Glu Cys Ile Gly Gly
   1 5 10 15
   Ser Gly Asp Gly
   20
<210> 50
   <211> 20
   <212> PRT
   <213> Artificial Sequence
<220>
   <223> aa503-522
<400> 50
   Asp Glu Cys Ile Gly Gly Ser Gly Asp Gly Met Ile Lys Val Lys Asn
   1 5 10 15
   Gln Leu Arg Phe
   20
<210> 51
   <211> 20
   <212> PRT
   <213> Artificial Sequence
<220>
   <223> aa513-532
<400> 51
   Met Ile Lys Val Lys Asn Gln Leu Arg Phe Leu Ala Glu Leu Ala Tyr
   1 5 10 15
   Asp Leu Asp Val
   20
<210> 52
   <211> 20
   <212> PRT
   <213> Artificial Sequence
<220>
   <223> aa523-542
<400> 52
   Leu Ala Glu Leu Ala Tyr Asp Leu Asp Val Asp Asp Ala Pro Gly Asn
   1 5 10 15
   Ser Gln Gln Ala
   20
<210> 53
   <211> 20
   <212> PRT
   <213> Artificial Sequence
<220>
   <223> aa533-552
<400> 53
   Asp Asp Ala Pro Gly Asn Ser Gln Gln Ala Thr Pro Lys Asp Asn Glu
   1               5                   10                  15
   Ile Ser Thr Phe
               20
<210> 54
   <211> 20
   <212> PRT
   <213> Artificial Sequence
<220>
   <223> aa543-562
<400> 54
   Thr Pro Lys Asp Asn Glu Ile Ser Thr Phe His Asn Leu Gly Asn Val
   1               5                   10                  15
   His Ser Pro Leu
               20
<210> 55
   <211> 20
   <212> PRT
   <213> Artificial Sequence
<220>
   <223> aa553-572
<400> 55
   His Asn Leu Gly Asn Val His Ser Pro Leu Lys Leu Leu Thr Ser Met
   1               5                   10                  15
   Ala Ile Ser Val
               20
<210> 56
   <211> 20
   <212> PRT
   <213> Artificial Sequence
<220>
   <223> aa561-580
<400> 56
   Pro Leu Lys Leu Leu Thr Ser Met Ala Ile Ser Val Val Cys Phe Phe
   1               5                   10                  15
   Phe Leu Val His
               20
<210> 57
   <211> 580
   <212> PRT
   <213> Homo sapiens
<400> 57

## Description

### TECHNICAL FIELD

The present invention relates to a method and use of a kit in the method for assisting recurrence risk prediction for hepatocellular carcinoma patients.

### BACKGROUND ART

Hepatocellular carcinoma is one of primary liver cancers and is a malignant tumor that arises from hepatocytes. In the treatment of hepatocellular carcinoma, hepatectomy to remove cancer and surrounding tissues by surgery or the like is performed. On the other hand, it is known that the recurrence rate of hepatocellular carcinoma patients as a whole is 70% or more in 3 years after surgery, even when treatment such as hepatectomy is performed. Therefore, hepatocellular carcinoma patients need to be examined continuously after surgery.

In the examination of hepatocellular carcinoma, measurement of a tumor marker in the blood and image inspection (ultrasonic inspection, CT, MRI, etc.) are performed in combination. Glypican 3 (GPC3) is known as a marker for hepatocellular carcinoma. GPC3 is a glycoprotein of about 60 kDa composed of 580 amino acids and is bound to a cell membrane via a glycosylphosphatidylinositol (GPI) anchor at the C-terminal region. GPC3 is expressed in 70 to 100% of liver tissues of hepatocellular carcinoma patients, but is hardly detected in liver tissues of healthy persons. It is considered that GPC3 is cleaved between R358/S359 by an enzyme called Furin in vivo and divided into an N-terminal peptide of about 40 kDa and a C-terminal peptide of about 30 kDa. Normally, it is considered that the N-terminal peptide and the C-terminal peptide are bound to the cell membrane in a state (full length) covalently bound by a disulfide bond.

Patent Literature 1 discloses a method of diagnosing cancer by detecting solubilized GPC3 using two types of antibodies that recognize the N-terminal peptide of GPC3. The inventors of Patent Literature 1 have concluded that the N-terminal peptide is superior as secretory GPC3 in mouse serum and it is preferable to measure GPC3 level using an anti-GPC3 antibody that recognizes the N-terminal peptide in the diagnosis of cancer. On the other hand, it has been suggested that it is difficult to diagnose cancer and predict recurrence risk by using a detection system using a C-terminal peptide recognition antibody (Table 1, paragraph 0138 and the like of Patent Literature 1). When two types of antibodies that recognize the N-terminal peptide of GPC3 are used, the N-terminal peptide of GPC3 and a full-length peptide are detected. However, in Patent Literature 1, since the full-length peptide is below the detection limit, it is considered that cancer determination has been made based on the measurement result of the N-terminal peptide.

Patent Literature 2 discloses a method for screening hepatocellular carcinoma (HCC) of a subject by measuring GPC3 level in a body fluid sample obtained from the subject using an anti-GPC3 monoclonal antibody that recognizes a C-terminal peptide of GPC3 and an anti-GPC3 polyclonal antibody that recognizes an arbitrary site in the amino acid sequence of GPC3. Patent Literature 2 does not mention the structure of GPC3 to be detected, but in view of the type of antibody used for GPC3 measurement, the C-terminal peptide of GPC3 and a full-length peptide are considered to be detected in the detection system described in the same literature. Incidentally, it is described in the paragraphs 0063 and 0064 of Patent Literature 2 that it is preferable to prepare polyclonal antibodies and monoclonal antibodies using the C-terminal peptide as an antigen. Furthermore, in Examples, polyclonal antibodies (Example 1) and monoclonal antibodies (Example 2) were prepared using the C-terminal peptide antigen, and the C-terminal peptide was detected using these antibodies. From these facts, in Patent Literature 2, the C-terminal peptide is considered to be detected. In fact, it is described in Patent Literature 1 that the full-length peptide is below the detection limit, and considering this, it is considered that cancer determination in Patent Literature 2 has been made based on the measurement result of the C-terminal peptide.
Kazuya Ofuji (2015 "Perioperative plasma glypican-3 level may enable prediction of the risk of recurrence after surgery in patients with stage I hepatocellular carcinoma" Hepatology 62:464A) discloses plasma GPC3 as a biomarker for predicting recurrence of hepatocellular carcinoma using an ELISA, without providing details regarding the reagents used for the ELISA, let alone the use of a pair of monoclonal antibodies against the N and C termini.

### CITATIONS LIST

### Patent Literatures

Patent Literature 1: US 20060014223 A
Patent Literature 2: US 20050233392 A

### SUMMARY OF INVENTION

### TECHNICAL PROBLEMS

Heretofore, a report supporting that measurement of blood GPC3 by a detection system using two types of N-terminal peptide recognition antibodies as described in Patent Literature 1 is useful for diagnosis and prognosis prediction of hepatocellular carcinoma has been made. However, in the prognosis prediction of hepatocellular carcinoma by such a report, it could not be said that the separation between the recurrence group and the non-recurrence group was good, and the determination accuracy was insufficient. On the other hand, in order to exert the physiological function (acceleration of cell proliferation, etc.) of GPC3, it is important that GPC3 is full length. In liver tissue staining of hepatocellular carcinoma patients, even when either the N-terminal peptide recognition antibody or the C-terminal peptide recognition antibody is used, the same result as when using both of them is obtained. Therefore, full-length GPC3 is present in liver tissue of hepatocellular carcinoma patient, and should be involved with formation of pathologic conditions. Furthermore, since GPC3 hardly exists in the liver of a healthy person, GPC3 should not be detected even in the blood. Nevertheless, blood GPC3 is also detected to some extent in healthy persons in the conventional detection system, and also from this point, the detection accuracy by the conventional detection system may be insufficient.

Based on these considerations, the present inventors considered that the full-length GPC3 in blood is an important factor for predicting poor prognosis, and in order to further improve the accuracy of diagnosis or prediction of recurrence risk of hepatocellular carcinoma, found a new problem of providing a new detection system using the full-length GPC3 as an object to be detected.

### SOLUTIONS TO PROBLEMS

As a result of intensive studies in order to solve the above problems, the present inventors have found that full-length GPC3 concentration in a blood sample can be measured by using a detection system using an N-terminal peptide-recognizing monoclonal antibody and a C-terminal peptide-recognizing monoclonal antibody, and that based on this, it is surprisingly possible to determine recurrence risk of hepatocellular carcinoma more accurately as compared to the conventional technique, and that full-length GPC3 is almost not detected in healthy persons by using this detection system, and thus have completed the present invention.

According to the present invention, there is provided a method for assisting prediction of recurrence risk for hepatocellular carcinoma patients, including steps of: measuring concentration of GPC3 peptide recognized by both a first monoclonal antibody and a second monoclonal antibody in a blood sample of hepatocellular carcinoma patient, using the first monoclonal antibody that recognizes a peptide having an amino acid sequence in the N-terminal side of GPC3 and the second monoclonal antibody that recognizes a peptide having an amino acid sequence in the C-terminal side of GPC3, wherein n the N- and C-terminal sides of GPC3 correspond respectively to the N- and C-terminal peptides obtained from cleavage of the full length GPC3 between the 358th and 359th amino acid; and determining that recurrence risk of hepatocellular carcinoma is high when the concentration of GPC3 peptide is equal to or greater than a predetermined threshold value, and determining that recurrence risk of hepatocellular carcinoma is low when the concentration of GPC3 peptide is less than the predetermined threshold value.

Furthermore, according to the present invention, there is provided use of a kit for assisting prediction of recurrence risk for hepatocellular carcinoma patients in a method according to the invention, said kit comprising a first monoclonal antibody that recognizes a peptide having an amino acid sequence in the N-terminal side of GPC3 and a second monoclonal antibody that recognizes a peptide having an amino acid sequence in the C-terminal side of GPC3, wherein the N- and C-terminal sides of GPC3 correspond respectively to the N- and C-terminal peptides obtained from cleavage of the full length GPC3 between the 358th and 359th amino acid.
More particularly, numbered statements of the present invention are:
1. A method for assisting prediction of recurrence risk for hepatocellular carcinoma patients, comprising steps of:
   measuring concentration of glypican 3 (GPC3) peptide recognized by both a first monoclonal antibody and a second monoclonal antibody in a blood sample of a hepatocellular carcinoma patient, using the first monoclonal antibody that recognizes a peptide having an amino acid sequence in the N-terminal side of GPC3 and the second monoclonal antibody that recognizes a peptide having an amino acid sequence in the C-terminal side of GPC3, wherein the N- and C-terminal sides of GPC3 correspond respectively to the N- and C-terminal peptides obtained from cleavage of the full length GPC3 between the 358th and 359th amino acid; and
   determining that recurrence risk of hepatocellular carcinoma is high when the concentration of GPC3 peptide is equal to or greater than a predetermined threshold value, and determining that recurrence risk of hepatocellular carcinoma is low when the concentration of GPC3 peptide is less than the predetermined threshold value.
2. The method according to statement 1, wherein the first monoclonal antibody is an antibody that recognizes a peptide having a sequence corresponding to the 301st to 358th amino acid of GPC3.
3. The method according to any one of statements 1 or 2, wherein the second monoclonal antibody is an antibody that recognizes a peptide having a sequence corresponding to the 543rd to 552nd amino acid of GPC3.
4. The method according to any one of statements 1 to 3, wherein the GPC3 peptide is a peptide containing the 301st to 552nd amino acid of GPC3.
5. The method according to any one of statements 1 to 4 wherein, in the determination step, among persons infected with hepatitis B virus having hepatocellular carcinoma, a GPC3-positive group is determined to have a high possibility of recurrence of hepatocellular carcinoma within a predetermined period.
6. The method according to any one of statements 1 to 5, wherein the measurement step is performed, in the presence of a non-specific reaction inhibitor.
7. Use of a kit comprising a first monoclonal antibody that recognizes a peptide having an amino acid sequence in the N-terminal side of GPC3 and a second monoclonal antibody that recognizes a peptide having an amino acid sequence in the C-terminal side of GPC3, , wherein the N- and C-terminal side of GPC3 correspond to the N- and C-terminal peptides obtained from cleavage of the full length GPC3 between the 358th and 359th amino acid, for assisting prediction of recurrence risk for hepatocellular carcinoma patients in a method according to any one of statements 1 to 6.

### ADVANTAGEOUS EFFECTS OF INVENTION

According to the present invention, it is possible to provide a method for assisting recurrence risk prediction for hepatocellular carcinoma patients, which can obtain a determination result with higher accuracy, as compared to the conventional technique.

### BRIEF DESCRIPTION OF DRAWINGS

Fig. 1 is a schematic diagram showing an example of a recurrence risk prediction assisting apparatus.
Fig. 2 is a block diagram showing a functional configuration of the recurrence risk prediction assisting apparatus in Fig. 1.
Fig. 3 is a block diagram showing a hardware configuration of the recurrence risk prediction assisting apparatus shown in Fig. 1.
Fig. 4A is an example of a flowchart of determination for acquiring data to assist recurrence risk prediction for hepatocellular carcinoma patients, using the recurrence risk prediction assisting apparatus shown in Fig. 1.
Fig. 4B is an example of a flowchart of determination for acquiring data to assist recurrence risk prediction for hepatocellular carcinoma patients, using the recurrence risk prediction assisting apparatus shown in Fig. 1.
Fig. 4C is an example of a flowchart of determination for acquiring data to assist recurrence risk prediction for hepatocellular carcinoma patients, using the recurrence risk prediction assisting apparatus shown in Fig. 1.
Fig. 5 is a diagram showing an example of an appearance of a kit.
Fig. 6 is a graph showing GPC3 concentration in blood samples of hepatocellular carcinoma patients measured in Example 1.
Fig. 7 is a Kaplan-Meier curve showing a classification result of recurrence risk stratified by the level of GPC3 concentration measured in Example 1.
Fig. 8 is a diagram illustrating epitope analysis performed in Example 3.
Fig. 9 is a diagram showing results of epitope analysis performed in Example 3.
Fig. 10 is a diagram showing results of epitope analysis performed in Example 3.
Fig. 11 is a diagram showing an epitope region of a C-terminal recognition type monoclonal antibody identified in Example 3.
Fig. 12 is a diagram illustrating epitope analysis of an N-terminal recognition type monoclonal antibody performed in Example 3.
Fig. 13 is a diagram showing results of epitope analysis of an N-terminal recognition type monoclonal antibody performed in Example 3.
Fig. 14 is a diagram showing an epitope region of an N-terminal recognition type monoclonal antibody identified in Example 3.
Fig. 15 is a graph showing a comparison of detection accuracy depending on whether a non-specific reaction inhibitor is added or not.
Fig. 16 is a graph showing a comparison of detection accuracy depending on whether a non-specific reaction inhibitor is added or not.
Fig. 17 is a diagram showing an analysis result based on the presence or absence of hepatitis B virus infection performed in Example 5.

### DESCRIPTION

A method for assisting prediction of recurrence risk for hepatocellular carcinoma patients of the present invention includes a step of measuring concentration of GPC3 peptide (hereinafter, also referred to as "GPC3 concentration") recognized by both a first monoclonal antibody and a second monoclonal antibody in blood samples of hepatocellular carcinoma patients, using the first monoclonal antibody that recognizes a peptide having an amino acid sequence in the N-terminal side of GPC3 and the second monoclonal antibody that recognizes a peptide having an amino acid sequence in the C-terminal side of GPC3, wherein the N- and C-terminal side of GPC3 correspond to the N- and C-terminal peptides obtained from cleavage of the full length GPC3 between the 358th and 359th amino acid.

Examples of the blood samples include blood (whole blood), plasma, serum, and the like obtained from hepatocellular carcinoma patients, and serum or plasma is preferably used. Alternatively, the blood sample may be a peptide solution obtained by pretreatment for extracting a peptide from blood, plasma or serum. The pretreatment method is not particularly limited, and a known method can be used. Further, the blood sample may be a diluted specimen obtained by diluting blood, plasma or serum with an appropriate aqueous medium. Such an aqueous medium is not particularly limited as long as it does not interfere with blood protein concentration measurement, and examples thereof include water, physiological saline, phosphate buffered saline (PBS), and the like. When measuring the GPC3 concentration by the immunoassay described later, the blood sample may be diluted with a blocking solution (a solution containing a blocking agent such as albumin or casein).

It is preferable that the blood sample be obtained from hepatocellular carcinoma patients before undergoing curative treatment. Here, examples of the curative treatment of hepatocellular carcinoma include hepatectomy, liver transplantation, percutaneous ethanol injection therapy, radiofrequency ablation therapy, heavy ion radiotherapy by proton beam and carbon beam, and the like. In addition, hepatocellular carcinoma patients may undergo auxiliary or preliminary treatment performed before the curative treatment in order to further improve the therapeutic effect of curative treatment of hepatocellular carcinoma. Examples of such auxiliary or preliminary treatment include hepatic arterial embolization, hepatic arterial infusion chemotherapy, immunotherapy such as vaccine, and the like. Therefore, blood samples collected from patients who have undergone hepatic arterial embolization, hepatic arterial infusion chemotherapy, immunotherapy such as vaccine, or the like as auxiliary or preliminary treatment, but have not undergone the curative treatment described above can be also used.

The method for measuring the GPC3 concentration is not particularly limited as long as it can acquire a value or index reflecting the GPC3 concentration in the blood sample, and can be appropriately selected from measurement methods known in the art. As such a measurement method, an immunoassay using an antigen-antibody reaction is preferable, and examples thereof include enzyme immunoassay, chemiluminescence immunoassay, fluorescence immunoassay, radioimmunoassay, immunoturbidimetry, immunoprecipitation, western blotting, affinity chromatography, and the like. Among them, an ELISA method of an enzyme immunoassay, particularly, a sandwich ELISA method is preferable.

The value of the GPC3 concentration may be the measured value itself or a value obtained by applying the measured value to a calibration curve. The calibration curve can be prepared from values obtained by preparing dilution series of GPC3 protein of known concentration and measuring them in the same manner as the blood sample. Based on this calibration curve, the GPC3 concentration in the sample can be quantitatively determined. The GPC3 protein for preparing a calibration curve may be a biological protein secreted by a hepatocarcinoma cell line such as HepG2 or may be a recombinant protein.

The first monoclonal antibody is not particularly limited as long as it recognizes a peptide having an amino acid sequence in the N-terminal side of GPC3. Here, an amino acid sequence in the N-terminal side of GPC3 refers to an amino acid sequence corresponding to the N-terminal side in the full-length GPC3 among GPC3 peptides obtained by cleavage between the 358th amino acid and 359th amino acid in full-length GPC3. The first monoclonal antibody is an antibody that recognizes a peptide having a sequence corresponding to the 1st to 358th amino acid of GPC3, and preferably an antibody that recognizes a peptide having a sequence corresponding to the 301st to 358th amino acid sequence of GPC3.

The second monoclonal antibody is not particularly limited as long as it recognizes a peptide having an amino acid sequence in the C-terminal side of GPC3. Here, an amino acid sequence in the C-terminal side of GPC3 refers to an amino acid sequence corresponding to the C-terminal side in the full-length GPC3 among GPC3 peptides obtained by cleavage between the 358th amino acid and 359th amino acid in the full-length GPC3. The second monoclonal antibody is an antibody that recognizes a peptide having a sequence corresponding to the 359th to 580th amino acid of GPC3, and preferably an antibody that recognizes a peptide having a sequence corresponding to the 543rd to 552nd amino acid of GPC3.

The origin of the first monoclonal antibody and the second monoclonal antibody is not particularly limited, and it may be an antibody derived from any mammal such as a mouse, a rat, a hamster, a rabbit, a goat, a horse, or a camel. Also, the isotype of the antibody is not particularly limited, and it may be appropriately selected according to the measurement method. The antibodies also include fragments of antibodies and derivatives thereof, and examples thereof include Fab fragments, F(ab')2 fragments, and the like.

Methods of preparing these antibodies and antibody fragments are known in the art. For example, the first monoclonal antibody can be prepared by immunizing a mouse with a peptide having only the amino acid sequence in the N-terminal side of GPC3, removing the spleen from the immunized mouse to obtain antibody-producing cells, fusing the obtained antibody-producing cells with tumor cells to form a hybridoma, and obtaining a monoclonal antibody produced from a suitably selected hybridoma, or the like. The second monoclonal antibody can also be prepared in the same manner, except for immunizing a mouse with using a peptide having only the amino acid sequence in the C-terminal side of GPC3. In addition, the first monoclonal antibody and the second monoclonal antibody may be commercially available products. Here, the amino acid sequence of the peptide antigen used for preparing the first monoclonal antibody is part or all of the amino acid sequence in the N-terminal side of GPC3. The amino acid sequence of the peptide antigen used for preparing the second monoclonal antibody is part or all of the amino acid sequence in the C-terminal side of GPC3.

In the method of the present invention, since the first monoclonal antibody and the second monoclonal antibody are used, GPC3 peptide recognized by both the first monoclonal antibody and the second monoclonal antibody in blood samples of hepatocellular carcinoma patients is the object to be detected. The forms of GPC3 which may be present in the blood sample include a peptide having the amino acid sequence in the N-terminal side of GPC3 but not having the amino acid sequence in the C-terminal side of GPC3 (N-terminal form, for example, a peptide having a 1st to 358th, 25th to 358th or 301st to 358th amino acid sequence of GPC3, or the like), a peptide having the amino acid sequence in the C-terminal side of GPC3 but not having the amino acid sequence in the N-terminal side of GPC3 (C-terminal form, for example, a peptide having a 359th to 580th, or 543th to 552nd amino acid sequence of GPC3, or the like) and a peptide having both the amino acid sequence in the N-terminal side of GPC3 and the amino acid sequence in the C-terminal side of GPC3 (full-length form, for example, a peptide having a 1st to 580th or 301st to 580th amino acid sequence of GPC3, or the like). Any conventional GPC3 detection systems as described in Patent Literatures 1 and 2 are not a method using the full-length form of GPC3 as the object to be detected. For example, in the method described in Patent Literature 1, cancer determination is made based on the measurement result of the N-terminal form of GPC3. In the method described in Patent Literature 2, cancer determination is made based on the measurement result of the C-terminal form of GPC3. In particular, as described in Patent Literature 1, it is recommended to detect the N-terminal form of GPC3 in diagnosis and prognosis prediction of hepatocellular carcinoma. In contrast, the present inventors used a full-length form of GPC3 as the object to be measured. The present inventors have found for the first time that detection accuracy is improved by using a detection system based on a full-length form of GPC3 peptide, more than when using a conventional detection system, and that recurrence risk for hepatocellular carcinoma patients can be predicted with high reliability. This is an unexpected observation by the present inventors, contrary to the knowledge in the conventional technical field.

In the measurement step, the GPC3 peptide to be detected is not particularly limited as long as it is recognized by both the first monoclonal antibody and the second monoclonal antibody. That is, the GPC3 peptide to be detected is a peptide having both an amino acid sequence in the N-terminal side of GPC3 and an amino acid sequence in the C-terminal side of GPC3, in other words, the full-length form of GPC3. For example, the GPC3 peptide to be detected may be a full-length protein containing a sequence corresponding to the 1st to 580th amino acid of GPC3 or may be a peptide containing a sequence corresponding to the 301st to 552nd amino acid sequence of GPC3. The amino acid sequence of the full-length GPC3 peptide is shown in SEQ ID NO: 57. A sugar chain, heparan sulfate or the like may be added to the GPC3 peptide to be detected. Since the GPC3 peptide to be detected is recognized by both the first monoclonal antibody and the second monoclonal antibody, it is considered that neither of the N-terminal form or the C-terminal form are substantially detected. The method of the present invention can substantially detect only the full-length form of GPC3, thereby contributing to high-accuracy recurrence risk prediction which could not be made with the conventional technique. It is also considered that components (N-terminal form, C-terminal form, other contaminants) other than the object to be detected are non-specifically detected by the first monoclonal antibody and the second monoclonal antibody. It is well known in the art that it is difficult to completely eliminate non-specific reactions that can be detected as background signals. The phrase "substantially detect only the full-length form of GPC3" means that non-specific detection of components other than the object to be detected may be included as long as the action and effect of the present invention are not lost.

The first monoclonal antibody and the second monoclonal antibody may be labeled with a labeling substance known in the art, as necessary. Examples of such a labeling substance include radioactive isotopes such as ³²P, ³⁵S, ³H and ¹²⁵I, fluorescent substances such as fluorescein, rhodamine and Alexa Fluor (registered trademark), enzymes such as alkaline phosphatase (ALP), horseradish peroxidase and luciferase, biotin, avidin, streptavidin, and the like.

In the case of using a solid phase for capturing antibodies in the measurement, the type of solid phase is not particularly limited. Examples of the solid phase include a solid phase of a material that physically adsorbs the antibody, a solid phase on which a molecule that specifically binds to the antibody is immobilized, and the like. Examples of the molecule that specifically binds to the antibody include protein A or G, an antibody (i.e., a secondary antibody) that specifically recognizes the antibody, and the like. In addition, a combination of substances interposed between the antibody and the solid phase can be used to bind them. Examples of such a combination of substances include combinations of biotin and avidin (or streptavidin), glutathione and glutathione-S-transferase, hapten and anti-hapten antibody, and the like. For example, when the first monoclonal antibody or the second monoclonal antibody is previously biotin-modified, the antibody can be captured by a solid phase on which avidin or streptavidin is immobilized.

The solid phase material can be selected from organic polymer compounds, inorganic compounds, biopolymers, and the like. Examples of the organic polymer compounds include latex, polystyrene, polypropylene, styrene-methacrylic acid copolymer, styrene-glycidyl (meth)acrylate copolymer, styrene-styrene sulfonate copolymer, methacrylic acid polymer, acrylic acid polymer, acrylonitrile butadiene styrene copolymer, vinyl chloride-acrylate copolymer, polyvinyl acetate acrylate, and the like. Examples of the inorganic compounds include magnetic bodies (iron oxide, chromium oxide, cobalt, ferrite, etc.), silica, alumina, glass, and the like. Examples of the biopolymer include insoluble agarose, insoluble dextran, gelatin, cellulose, and the like. Two or more of these may be used in combination. The shape of the solid phase is not particularly limited, and examples thereof include particles, microplates, microtubes, test tubes, and the like.

The measurement step is preferably performed in the presence of a non-specific reaction inhibitor. The presence of a non-specific reaction inhibitor makes it possible to reduce the possibility of being determined to be false positive.

The non-specific reaction inhibitor is not particularly limited as long as it suppresses binding of the antibody used in the measurement to a substance other than the target antigen. Specific examples include mouse serum, sheep serum, goat serum, mouse antibody and sheep antibody, goat antibody, and the like. Non-specific reaction inhibitors are commercially available, and, for example, TRUBlock (Meridian Life Science, Inc.), ASSAY DEVELOPMENT BLOCKING KIT (SCANTIBODIS LABORATORY, Inc.), THBR2 (Institute of Immunology Co., Ltd.), and the like are known.

The method of the present invention includes a step of determining that recurrence risk of hepatocellular carcinoma is high when the concentration of GPC3 peptide is equal to or greater than a predetermined threshold value, and determining that recurrence risk of hepatocellular carcinoma is low when the concentration of GPC3 peptide is less than the predetermined threshold value (hereinafter, also simply referred to as "determination step"). In the present specification, it is sometimes referred to as GPC3 positive when the concentration of GPC3 peptide is equal to or greater than the threshold value, and as GPC3 negative when the concentration is less than the threshold value.

Here, recurrence refers to that patients who have undergone curative treatment for hepatocellular carcinoma again suffer from hepatocellular carcinoma. The phrase "recurrence risk is high" means that the possibility that hepatocellular carcinoma patients who have undergone curative treatment again suffer from hepatocellular carcinoma from the curative treatment is high. On the other hand, the phrase "recurrence risk is low" means that the possibility that hepatocellular carcinoma patients who have undergone curative treatment again suffer from hepatocellular carcinoma from the curative treatment is low.

The threshold value is not particularly limited, and can be appropriately set in advance by a person skilled in the art. For example, the concentrations of the GPC3 full length form of a plurality of hepatocellular carcinoma patients (non-recurrent patient group) who have not recurred for a certain period (e.g., 3 years, 4 years, or 5 years) after surgery and a plurality of hepatocellular carcinoma patients (recurrent patient group) who have recurred after surgery are measured in advance, and the threshold value can be set to a value that can most accurately classify the non-recurrent patient group and the recurrent patient group. When setting the threshold value, it can be set in consideration of sensitivity, specificity, positive predictive value, negative predictive value, and the like, according to the purpose of the examination. In addition, the threshold value may be set as follows:
1) First, GPC3 concentration is measured for each of the blood samples obtained before surgery from the non-recurrent patient group and blood samples obtained before surgery from the recurrent patient group.
2) Based on the measurement result, the threshold value is set from a range that is higher than the GPC3 concentration value of the non-recurrent patients and lower than the GPC3 concentration value of the recurrent patients.

Preferably, a value that can highly accurately distinguish the non-recurrent patients and the patients with cancer recurrence is set as the threshold value. In the examples described later, the threshold value of GPC3 concentration is set to 15 pg/mL. In the present embodiment, the concentration of GPC3 peptide in blood samples of hepatocellular carcinoma patients is compared with the threshold value preset as described above, and into which of the non-recurrent patient group and the recurrent patient group it is classified is predicted. As a result of the examination, for example, a determination result that the recurrence risk is low is output when it is classified as the non-recurrent patient group, and a determination result that the recurrence risk is high is output when it is classified as the recurrent patient group.

Alternatively, GPC3 concentration data is accumulated for a person who is not a hepatocellular carcinoma patient, for example, a healthy person or a patient who has a disease other than hepatocellular carcinoma, and reference values (for example, average value, median value, etc.) of the blood concentration of GPC3 are determined based on the data, and this reference value may be used as the threshold value.

In a further embodiment, a method for assisting prediction of recurrence risk for hepatocellular carcinoma patients, when a patient who is determined to have a high recurrence risk as described above is further infected with hepatitis B virus, the patient is determined to have a high possibility of recurrence of hepatocellular carcinoma earlier than a GPC3-negative patient. That is, in hepatocellular carcinoma patients infected with hepatitis B virus, not only the presence or absence of recurrence but also the time of recurrence can be predicted based on the presence or absence of GPC3 detection.

The hepatitis B virus (Species: Hepatitis B virus; HBV) means a kind of virus belonging to Order: Orthohepadnavirus, Family: Hepadnaviridae, with reference to the virus taxonomy published in 2015 by the International Committee on Taxonomy of Viruses (ICTV). In the virus taxonomy, reorganization of taxonomy and modification of genus names and the like are frequently done. Therefore, even when taxonomy is reorganized in the future in taxonomy by ICTV or equivalent academic authority organization, or genus names and the like are modified, the virus corresponding to hepatitis B virus in the ICTV taxonomy in 2015 is included in the hepatitis B virus referred to in the present specification.

The genotype of hepatitis B virus is not particularly limited, and may be any of genotypes A to J known at the filing date of the present application and their subtypes (for example, Aa/1, etc.), or may be a genotype unknown at the filing date of the present application, which may be found in the future.

Infection means that there is at least a detectable level of hepatitis B virus in the patient's body fluid, especially blood.

When whether or not a subject is infected with hepatitis B virus is not known, the diagnosis assisting method may further include a step of detecting hepatitis B virus in the blood samples of hepatocellular carcinoma patients. Whether or not the subject is infected with hepatitis B virus can be examined by a virus detection method known to a person skilled in the art. Examples of such detection method include an ELISA method of an enzyme immunoassay, a direct sequence method, and the like.

On the other hand, when it is known in advance that a subject is infected with hepatitis B virus, it is not necessary to perform the step of detecting hepatitis B virus. For example, when a patient who was diagnosed as suffering from hepatitis B and whose disease has then progressed to hepatocellular carcinoma is a subject, it is known that the subject is infected with hepatitis B virus, and thus it is not necessary to perform the step of detecting hepatitis B virus.

Further disclosed herein is a method for assisting prediction of recurrence risk for hepatocellular carcinoma patients, wherein blood samples of persons infected with hepatitis B virus having hepatocellular carcinoma are used. In said method, the concentration of GPC3 peptide is measured in the same manner as described above and compared with a predetermined threshold value, whereby the infected persons are classified into a GPC3-positive group (first group) in which the concentration of GPC3 peptide is equal to or greater than the predetermined threshold value or a GPC3-negative group (second group) in which the concentration of GPC3 peptide is less than the predetermined threshold value. The GPC3-positive group is determined to have a high possibility of having a short recurrence-free period of hepatocellular carcinoma (recurring early) as compared to the GPC3-negative group. The GPC3-negative group is determined to have a high possibility of having a recurrence-free period of hepatocellular carcinoma longer than the GPC3-positive group or having no recurrence of hepatocellular carcinoma.

In embodiments of the method of the invention, among persons infected with hepatitis B virus having hepatocellular carcinoma, the GPC3-positive group is determined to have a high possibility of recurrence of hepatocellular carcinoma within a predetermined period. The predetermined period can be set as a period during which the GPC3-positive group and the GPC3-negative group can be most accurately separated, based on the information on the recurrence-free period of the GPC3-positive group and the GPC3-negative group. For example, the predetermined period can be set as 4 months, 5 months, 6 months, 7 months, 8 months, 9 months, 10 months, 11 months, or 12 months.

Further disclosed herein is a method of treating hepatocellular carcinoma. This method includes: a step of performing curative treatment for a patient determined to "have a high recurrence risk" by the determination step described above; and a step of administering a drug that reduces recurrence risk of hepatocellular carcinoma after curative treatment. The curative treatment is as described above. As a drug that reduces recurrence risk, known drugs can be used, and examples thereof include antiviral drugs such as interferon and the like.

Also disclosed herein is an apparatus suitable for acquiring data to assist prediction of recurrence risk for hepatocellular carcinoma patients. Also disclosed herein is a computer program product for making a computer acquire data to assist prediction of recurrence risk for hepatocellular carcinoma patients. The medium included in the computer program product may be a computer-readable medium in which a computer program is non-temporarily recorded.

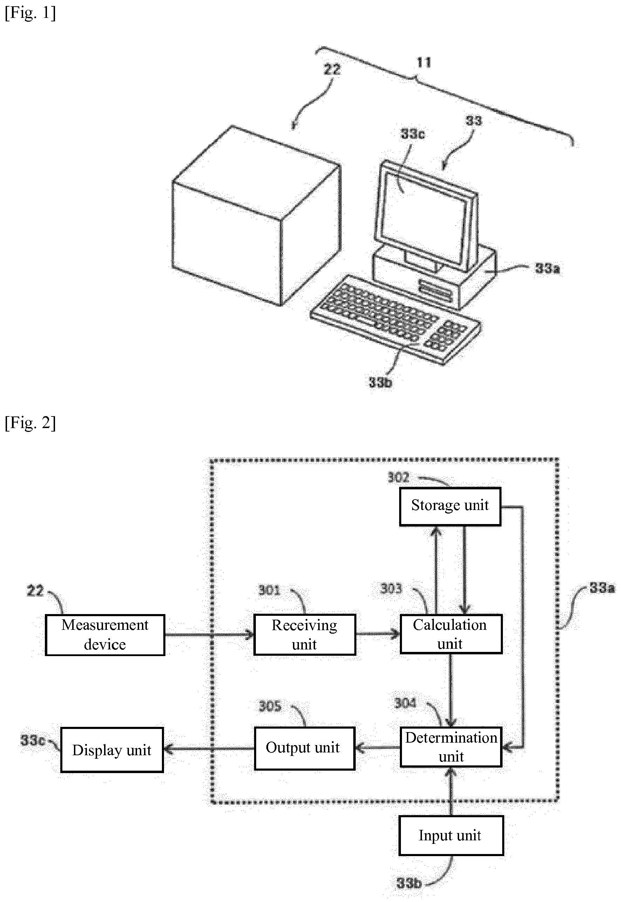

Hereinafter, an example of an apparatus suitable for implementing the above-described method of the present invention will be described with reference to the drawings. Fig. 1 is a schematic diagram of a recurrence risk prediction assisting apparatus for acquiring data to assist prediction of recurrence risk for hepatocellular carcinoma patients. A recurrence risk prediction assisting apparatus 11 shown in Fig. 1 includes a measurement device 22, and a determination device 33 connected to the measurement device 22.

The type of measurement device is not particularly limited, and it can be appropriately selected according to the method of measuring GPC3. In the example shown in Fig. 1, the measurement device 22 is a plate reader capable of detecting a signal generated by an ELISA method using a labeled antibody. The plate reader is not particularly limited as long as it can detect a signal based on the labeling substance used for measuring the marker, and it can be appropriately selected according to the type of the labeling substance. In the example described below, the signal is optical information such as a chemiluminescent signal or a fluorescence signal.

When the plate subjected to an antigen-antibody reaction is set in the measurement device 22, the measurement device 22 acquires optical information based on the labeled antibody specifically bound to GPC3, and transmits the obtained optical information to the determination device 33. The optical information acquired by the measurement device 22 also includes information on a specimen whose concentration is known for calculation of a concentration value. Further, in the example described below, the measurement device 22 can also be used for detection of hepatitis B virus by selecting an appropriate labeled antibody.

The determination device 33 includes a computer main body 33a, an input unit 33b, and a display unit 33c that displays specimen information, determination results, and the like. The determination device 33 receives the optical information from the measurement device 22. Then, a processor of the determination device 33 executes a program for acquiring data to assist prediction of recurrence risk for hepatocellular carcinoma patients, based on the optical information. It is possible to input the phrase "infected with hepatitis B virus" or "necessary to detect hepatitis B virus" to be described later from the input unit 33b.

Fig. 2 is a block diagram showing software of the computer main body 33a of the recurrence risk prediction assisting apparatus 11 by functional blocks. As shown in Fig. 2, the determination device 33 includes a receiving unit 301, a storage unit 302, a calculation unit 303, a determination unit 304, and an output unit 305. The receiving unit 301 is communicably connected to the measurement device 22 via a network. It is possible to input information for implementing recurrence risk prediction of hepatocellular carcinoma, for example, information on whether or not a specimen determined to have a high risk is infected with hepatitis B virus, or whether or not detection of hepatitis B virus is performed, to the determination unit 304 via the input unit 33b.

The receiving unit 301 receives the information transmitted from the measurement device 22. The storage unit 302 stores an expression for calculating a threshold value required for determination and a concentration value of each marker, a processing program, and when hepatitis B virus detection is performed, further stores optical information (signals, etc.) obtained when infected and/or not infected with hepatitis B virus in an antigen-antibody reaction, and the like. Using the information acquired by the receiving unit 301, the calculation unit 303 calculates a concentration value of each marker, according to the expression stored in the storage unit 302. The determination unit 304 determines whether the concentration value of the marker calculated by the calculation unit 303 is equal to or greater than or less than the threshold value stored in the storage unit 302, and when hepatitis B virus detection is performed, further determines the presence or absence of hepatitis B virus infection, based on whether or not the optical information acquired from the measurement device 22 in the receiving unit 301 coincides with that stored in the storage unit 302. The output unit 305 outputs the determination result of the determination unit 304 as data for assisting prediction of recurrence risk for hepatocellular carcinoma patients.

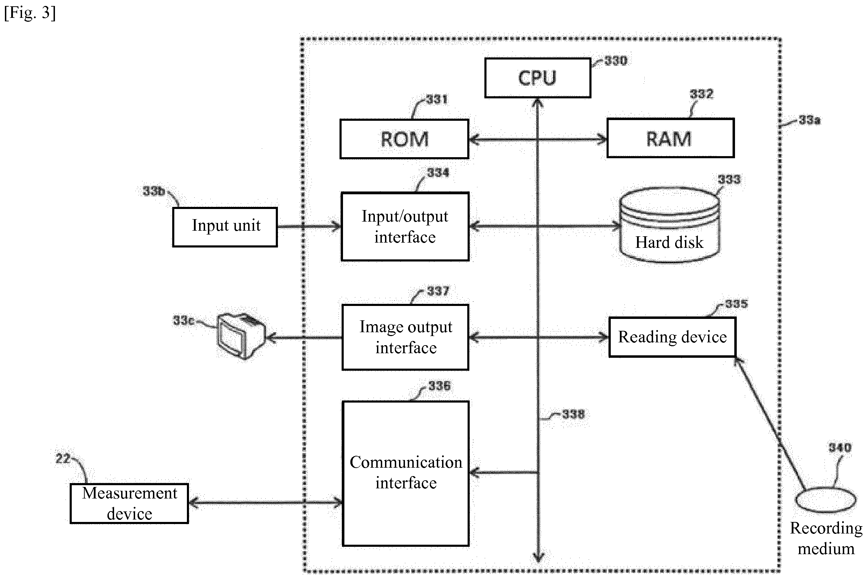

Fig. 3 is a block diagram showing a hardware configuration of the computer main body 33a shown in Fig. 2. As shown in Fig. 3, the computer main body 33a includes a CPU (Central Processing Unit) 330, a ROM (Read Only Memory) 331, a RAM (Random Access Memory) 332, a hard disk 333, an input/output interface 334, a reading device 335, a communication interface 336, and an image output interface 337. The CPU 330, the ROM 331, the RAM 332, the hard disk 333, the input/output interface 334, the reading device 335, the communication interface 336 and the image output interface 337 are data-communicably connected by a bus 338.

The CPU 330 can execute a computer program stored in the ROM 331 and a computer program loaded in the RAM 332. Each function shown in Fig. 2 is executed by the CPU 330 executing an application program. Accordingly, the determination device 33 functions as a device for acquiring data for assisting prediction of recurrence risk for hepatocellular carcinoma patients.

The ROM 331 is constituted of a mask ROM, a PROM, an EPROM, an EEPROM, and the like. In the ROM 331, a computer program executed by the CPU 330 and data used therefor are recorded.

The RAM 332 is constituted of an SRAM, a DRAM, and the like. The RAM 332 is used for reading the computer program recorded in the ROM 331 and the hard disk 333. The RAM 332 is also used as a work area of the CPU 330 when executing these computer programs.

The hard disk 333 has installed therein an operating system for making the CPU 330 execute, a computer program such as an application program (a computer program for acquiring data to assist prediction of recurrence risk for hepatocellular carcinoma patients) and data used for executing the computer program.

The reading device 335 is constituted of a flexible disk drive, a CD-ROM drive, a DVD-ROM drive, and the like. The reading device 335 can read a computer program or data recorded on a portable recording medium 340.

The input/output interface 334 is constituted of, for example, a serial interface such as USB, IEEE1394 or RS-232C, a parallel interface such as SCSI, IDE or IEEE1284, and an analog interface including a D/A converter, an A/D converter and the like. The input unit 33b such as a keyboard and a mouse are connected to the input/output interface 334. An operator can input various commands and data to the computer main body 33a through the input unit 33b.

The communication interface 336 is, for example, an Ethernet (registered trademark) interface or the like. The computer main body 33a can transmit print data to a printer or the like through the communication interface 336. The measurement device 22 can transmit measurement data (optical information) to the determination device 33 through the communication interface 336.

The image output interface 337 is connected to the display unit 33c constituted of an LCD, a CRT, and the like. Accordingly, the display unit 33c can output the video signal corresponding to image data given from the CPU 330. The display unit 33c displays an image (screen) according to the input video signal.

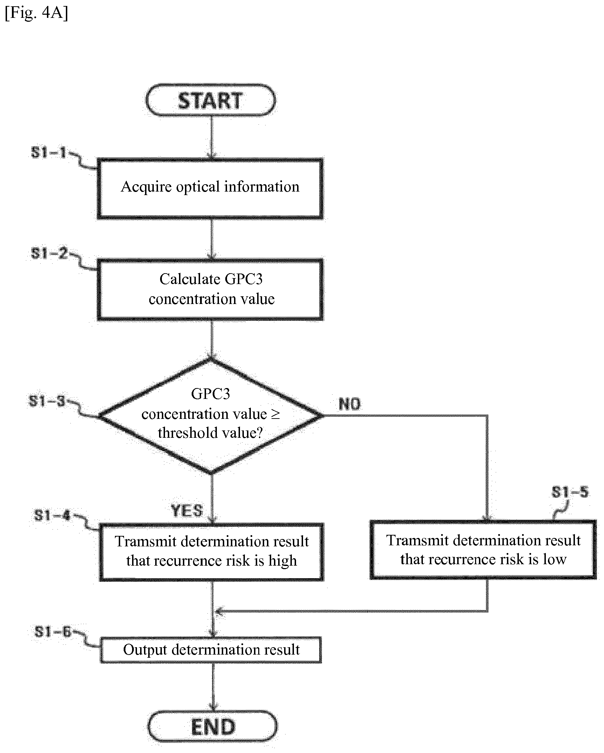

Next, a processing procedure for acquiring data to assist prediction of recurrence risk for hepatocellular carcinoma patients by the recurrence risk prediction assisting apparatus 11 will be described. Fig. 4A is an example of a flowchart for acquiring data to assist recurrence risk prediction. A case where a concentration value of GPC3 is acquired from the optical information based on the labeled antibody specifically bound to GPC3 and a determination is made using the obtained concentration value will be described as an example.

First, in Step S1-1, the receiving unit 301 of the recurrence risk prediction assisting apparatus 11 receives optical information from the measurement device 22. Next, in Step S1-2, the calculation unit 303 calculates a concentration value of GPC3, according to the expression for calculating a concentration value stored in the storage unit 302, from the optical information received by the receiving unit 301.

Thereafter, in Step S1-3, the determination unit 304 determines whether the concentration value calculated in Step S1-2 is equal to or greater than or less than the threshold value stored in the storage unit 302. Here, when the concentration value of GPC3 is equal to or greater than the threshold value, the routine proceeds to Step S1-4, and the determination unit 304 transmits to the output unit 305 a determination result indicating that the recurrence risk for hepatocellular carcinoma patients is high. When the concentration value of GPC3 is less than the threshold value, the routine proceeds to Step S1-5, and the determination unit 304 transmits to the output unit 305 a determination result indicating that the recurrence risk for hepatocellular carcinoma patients is low.

Then, in Step S1-6, the output unit 305 outputs the determination result. The output unit 305 displays it on the display unit 33c. Accordingly, the recurrence risk prediction assisting apparatus 11 can provide doctors and the like with data to assist prediction of recurrence risk for hepatocellular carcinoma patients.

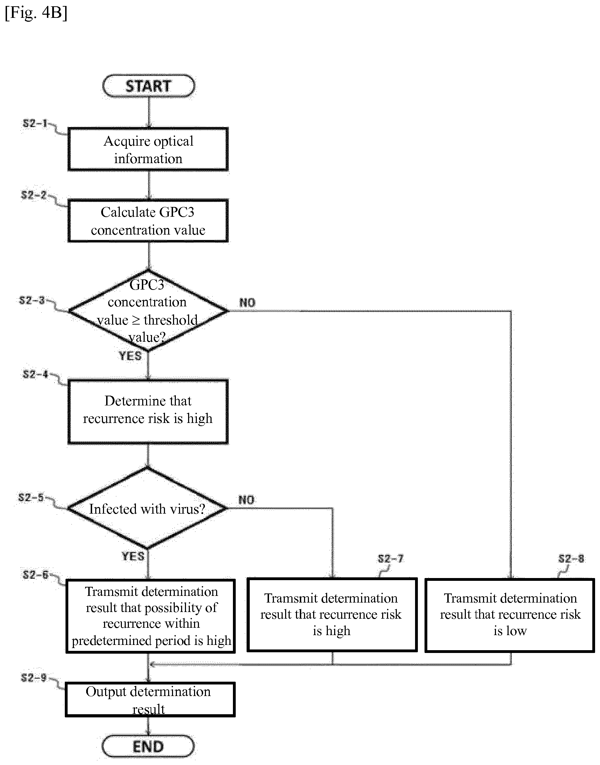

Fig. 4B is an example of a flowchart for acquiring data to assist recurrence risk prediction, which is performed on a specimen known to be infected with hepatitis B virus.

First, in Step S2-1, the receiving unit 301 of the recurrence risk prediction assisting apparatus 11 receives optical information from the measurement device 22. Next, in Step 2-2, the calculation unit 303 calculates a concentration value of GPC3, according to the expression for calculating a concentration value stored in the storage unit 302, from the optical information received by the receiving unit 301.

Thereafter, in Step S2-3, the determination unit 304 determines whether the concentration value calculated in Step S2-2 is equal to or greater than or less than the threshold value stored in the storage unit 302. When the concentration value of GPC3 is less than the threshold value, the routine proceeds to Step S2-8, and the determination unit 304 transmits to the output unit 305 a determination result indicating that the recurrence risk for hepatocellular carcinoma patients is low.

On the other hand, in Step S2-3, when the concentration value of GPC3 is equal to or greater than the threshold value, the routine proceeds to Step S2-4, the determination unit 304 determines that the recurrence risk for hepatocellular carcinoma patients is high, and then the routine proceeds to Step S2-5.

In Step S2-5, when the phrase "infected with hepatitis B virus" is input from the input unit 33b, the routine proceeds to Step S2-6, and the determination unit 304 transmits to the output unit 305 a determination result indicating that the possibility of recurrence of hepatocellular carcinoma within a predetermined period is high. On the other hand, in Step S2-5, when the phrase "infected with hepatitis B virus" is not input from the input unit 33b, the routine proceeds to Step S2-7, and the determination unit 304 transmits to the output unit 305 a determination result indicating that the recurrence risk for hepatocellular carcinoma patients is high.

Then, in Step S2-9, the output unit 305 outputs the determination result. The output unit 305 displays it on the display unit 33c. Accordingly, the recurrence risk prediction assisting apparatus 11 can provide doctors and the like with data to assist prediction of recurrence risk for hepatocellular carcinoma patients.

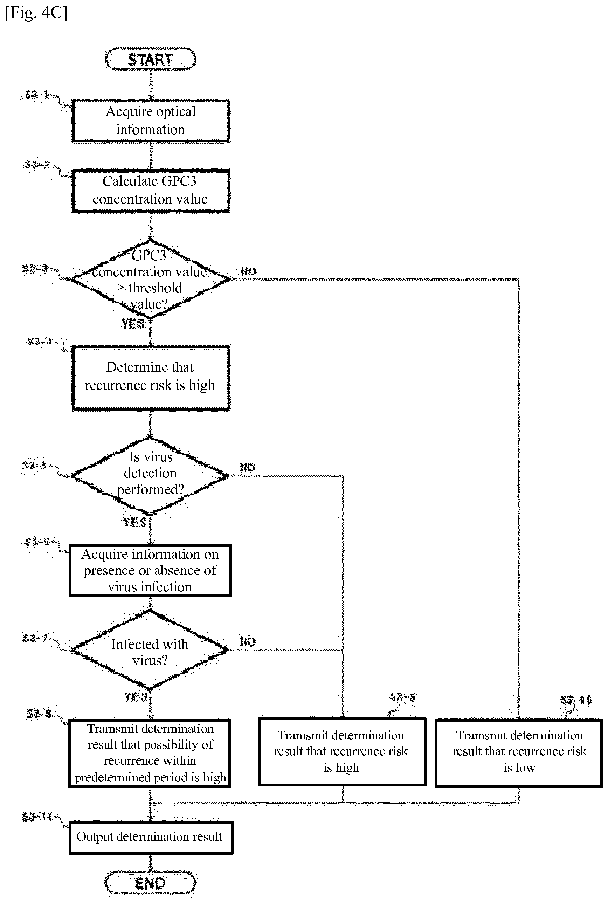

Fig. 4C is an example of a flowchart for acquiring data to assist recurrence risk prediction when the detection step of hepatitis B virus is performed. First, in Step S3-1, the receiving unit 301 of the recurrence risk prediction assisting apparatus 11 receives optical information from the measurement device 22. Next, in Step S3-2, the calculation unit 303 calculates a concentration value of GPC3, according to the expression for calculating a concentration value stored in the storage unit 302, from the optical information received by the receiving unit 301.

Thereafter, in Step S3-3, the determination unit 304 determines whether the concentration value calculated in Step S3-2 is equal to or greater than or less than the threshold value stored in the storage unit 302. When the concentration value of GPC3 is less than the threshold value, the routine proceeds to Step S3-10, and the determination unit 304 transmits to the output unit 305 a determination result indicating that the recurrence risk for hepatocellular carcinoma patients is low.

On the other hand, in Step S3-3, when the concentration value of GPC3 is equal to or greater than the threshold value, the routine proceeds to Step S3-4, the determination unit 304 determines that the recurrence risk for hepatocellular carcinoma patients is high, and then the routine proceeds to Step S3-5. Then, in Step S3-5, when the phrase "necessary to detect hepatitis B virus" is not input from the input unit 33b, the routine proceeds to Step S3-9, and the determination unit 304 transmits to the output unit 305 a determination result indicating that the recurrence risk for hepatocellular carcinoma patients is high.

On the other hand, when the phrase "necessary to detect hepatitis B virus" is input from the input unit 33b, the routine proceeds to Step S3-6, and the receiving unit 301 receives information on the presence or absence of hepatitis B virus infection, from the measurement device 22. Thereafter, the routine proceeds to Step S3-7, and the determination unit 304 determines whether or not the patient is infected with hepatitis B virus, based on the information acquired in Step S3-6. When it is determined that the patient is infected with hepatitis B virus, the routine proceeds to Step S3-8, and the determination unit 304 transmits to the output unit 305 a determination result indicating that the possibility of recurrence of hepatocellular carcinoma within a predetermined period is high. On the other hand, when it is not determined that the patient is infected with hepatitis B virus, the routine proceeds to Step S3-9, and the determination unit 304 transmits to the output unit 305 a determination result indicating that the recurrence risk of hepatocellular carcinoma is high.

Then, in Step S3-11, the output unit 305 outputs the determination result. The output unit 305 displays it on the display unit 33c. Accordingly, the recurrence risk prediction assisting apparatus 11 can provide doctors and the like with data to assist prediction of recurrence risk for hepatocellular carcinoma patients.

The present invention also includes use of a kit for assisting prediction of recurrence risk for hepatocellular carcinoma patients in a method according to the invention, including a first monoclonal antibody that recognizes a peptide having an amino acid sequence in the N-terminal side of GPC3 and a second monoclonal antibody that recognizes a peptide having an amino acid sequence in the C-terminal side of GPC3, wherein the N- and C-terminal side of GPC3 correspond to the N- and C-terminal peptides obtained from cleavage of the full length GPC3 between the 358th and 359th amino acid. The first monoclonal antibody and the second monoclonal antibody are as described above. The kit may further include the above-mentioned non-specific reaction inhibitor. Further, the kit may further include the above-mentioned solid phase, for example, a 96-well plate or magnetic particles.

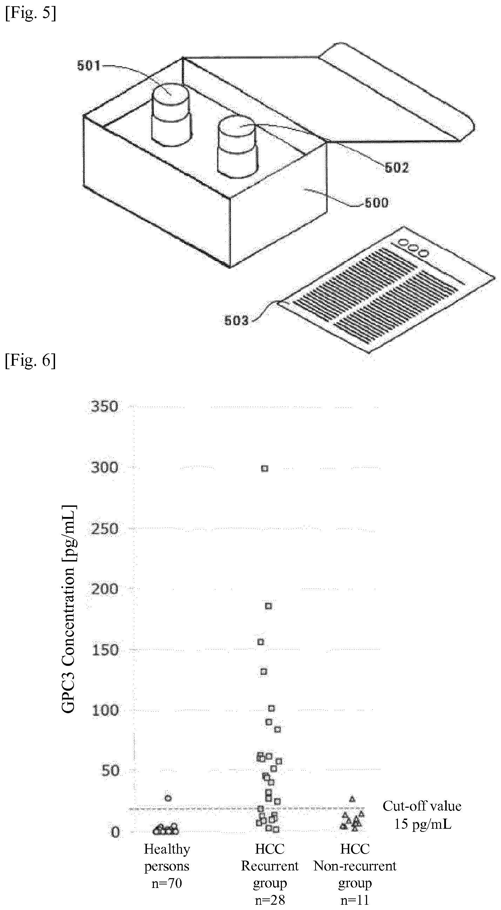

For example, as shown in Fig. 5, the kit may include a first reagent container 501 storing a first reagent containing a first monoclonal antibody that recognizes a peptide having an amino acid sequence in the N-terminal side of GPC3, and a second reagent container 502 storing a second reagent containing a second monoclonal antibody that recognizes a peptide having an amino acid sequence in the C-terminal side of GPC3. The kit may further include a package insert 503 describing the above method and a box 500 storing the first reagent container 501, the second reagent container 502, and the reagent package insert 503.

In addition, the kit may further include a reagent containing a substance for detecting hepatitis B virus. The substance for detecting hepatitis B virus is not particularly limited and can be appropriately selected depending on the type of a method for detecting hepatitis B virus. Examples of such a substance include an antibody that specifically recognizes an envelope or the like of hepatitis B virus, a primer suitable for amplifying a genomic DNA of hepatitis B virus, and the like.

Further disclosed herein is use of a first monoclonal antibody and a second monoclonal antibody for producing a GPC3 peptide detection reagent kit. Also disclosed is use of a substance for detecting hepatitis B virus for producing a GPC3 peptide detection reagent kit. The first monoclonal antibody, the second monoclonal antibody, and the substance and kit for detecting hepatitis B virus are as described above.

Hereinafter, the present invention will be described in detail with reference to examples, but is not limited to these examples.

### EXAMPLES

### Example 1: Recurrence Risk Prediction

Thirty-nine HCC patient blood samples were collected before hepatectomy treatment at the National Cancer Center Hospital East (Kashiwa City, Chiba Prefecture) with consent from patients, and plasma was obtained by centrifugation. Recurrence of HCC was confirmed within 4 years after hepatectomy treatment in 28 cases among them, and 11 cases were non-recurrent.

Fifty healthy person plasma samples were purchased from SUNFCO LTD. All samples were cryopreserved at -80°C until just before use.

Measurement of GPC3 in each patient and healthy person plasma samples was performed as follows. First, a 96-well immunoplate (MaxiSorp black 96-well immunoplate, Thermo Fisher Scientific Inc.) was coated with 50 µL each of a 2 µg/mL commercially available GPC3 N-terminal recognizing monoclonal antibody diluted with phosphate buffered saline (PBS), and the plate was incubated at room temperature for 1 hour. Next, the plate was washed nine times with 300 µL of HISCL washing solution (Sysmex Corporation), then added with 250 µL of blocking buffer (buffer at pH 7.4 containing 150 mM NaCl, 10 mg/mL BSA, 5 mg/mL Casein, 0.01 w/v% NaN₃), and the plate was incubated at room temperature for 2 hours. Thereafter, the plate was washed nine times with 300 µL of HISCL washing solution, then 50 µL of a measurement sample diluted 2-fold with dilution buffer prepared by adding non-specific reaction inhibitor for HISCL (Sysmex Corporation) to blocking buffer was added, and the plate was incubated at room temperature for 1 hour. Subsequently, the plate was washed nine times with 300 µL of HISCL washing solution, and a commercially available GPC3 C-terminal recognizing monoclonal antibody labeled according to the attached protocol using Alkaline Phosphatase Labeling Kit-SH (Dojindo Laboratories) was diluted to a final concentration of 0.1 µg/mL with dilution buffer, then 50 µL each of the dilution was added to the plate, and the plate was incubated at room temperature for 1 hour. Thereafter, the plate was washed nine times with 300 µL of HISCL washing solution, 100 µL each of HISCL luminescent substrate set (Sysmex Corporation) was added to thereto, the plate was incubated at room temperature in a dark place for 30 minutes, and then the luminescence intensity was measured with a microplate reader (Infinite F200 PRO, Tecan Japan Co., Ltd.).

As a standard sample for preparing a calibration curve, one obtained by diluting a full-length recombinant GPC3 (R&D Systems, Inc.) with blocking buffer so as to be 15 to 20,000 pg/mL was used. In addition, a calibration curve was prepared using 4 parameter logistic regression. After all the measurement was completed, the GPC3 concentration in the measurement sample was quantitatively determined from the luminescence intensity using the calibration curve.

For the obtained measurement results, positive/negative determination was made with a threshold value of 15 pg/mL. As shown in Fig. 6 and Table 1, the positive rate of 28 recurrent cases was 71.4% (20/28 cases), whereas the positive rate of 11 non-recurrent cases was 9.1% (1/11 cases), and it was found that the determination efficiency of non-recurrence improved as compared to other existing HCC markers (AFP and PIVKA-II). In addition, as shown in Fig. 6, almost no GPC3 was detected in the healthy persons. This is consistent with the knowledge that GPC3 is hardly detected in liver tissues of healthy persons. Therefore, it can be said that the detection accuracy of GPC3 is extremely high in the method of this embodiment.

**[Table 1]**

| | Recurrent group | Non-recurrent group |
|---|---|---|
| Full-length GPC3 | 71.4% | 9.1% |
| | (20/28) | (1/11) |
| AFP | 60.7% | 27.3% |
| | (17/28) | (3/11) |
| PIVKA-II | 71.4% | 72.7% |
| | (20/28) | (8/11) |

### Example 2: Recurrence Risk Prediction Using Kaplan-Meier

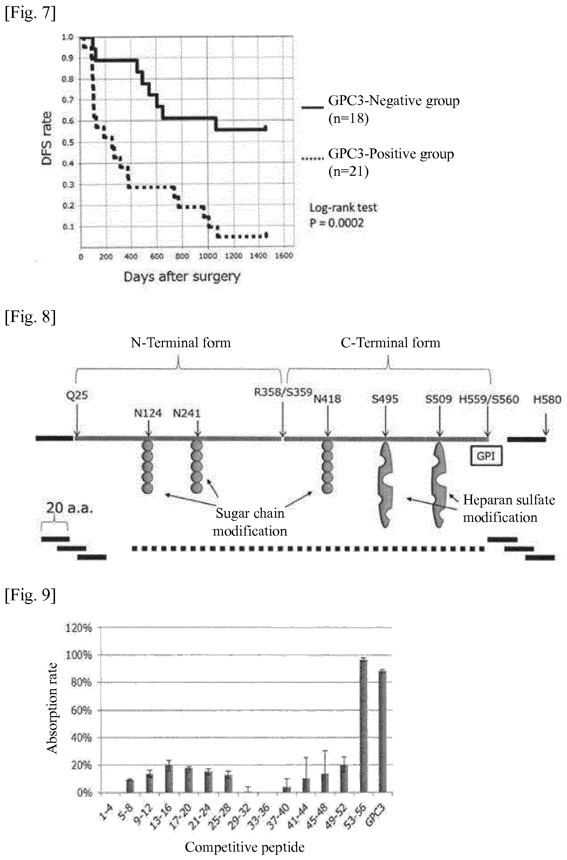

Furthermore, the GPC3-positive/negative group determined by the measurement result obtained in Example 1 was tested for a significant difference in recurrence risk prediction. A Kaplan-Meier curve was prepared for the presence or absence of recurrence and a log rank test was performed. As a result, as shown in Fig. 7, it was shown that the P value was 0.0002 and the GPC3-positive group significantly tended to recur. For statistical analysis software, StatFlex ver. 6 (Artec Co., Ltd.) was used.

### Example 3: Epitope Analysis of Antibody Used This Time

The epitopes of the antibodies used in Examples 1 and 2 were analyzed to verify whether the reason why the recurrence risk determination accuracy of HCC was dramatically improved is to correctly grasp full-length GPC3.

Epitope analysis of the C-terminal recognition type monoclonal antibody used for detection was performed by competitive ELISA using overlapping peptides (Table 2) as shown in Fig. 8.

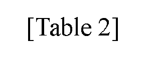

35 µL of a solution obtained by diluting a commercially available C-term

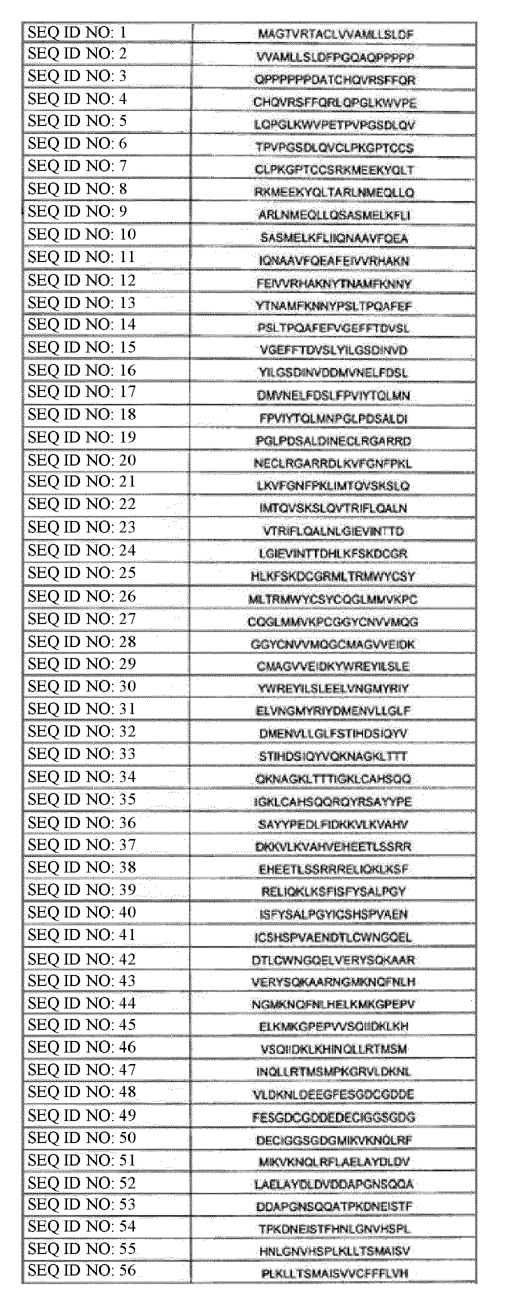

inal recognition type monoclonal antibody with blocking buffer (buffer at pH 7.0 containing 150 mM NaCl, 2.5 mM EDTA, 0.1 mg/mL BSA) so as to have a final concentration of 0.2 µg/mL was mixed with a competitive peptide prepared in blocking buffer to have a 10 times concentration of the antibody in molar ratio or 35 µL of a full-length recombinant GPC3 solution, and the mixture was incubated at room temperature for 1 hour to obtain a competitive inhibition solution. In addition, 50 µL each of full-length recombinant GPC3 diluted with PBS so as to be 10 ng/mL was added to a 96-well immunoplate (MaxiSorp white 96-well immunoplate, Thermo Fisher Scientific Inc.), and the plate was incubated at room temperature for 1 hour. The plate was washed three times with 300 µL of HISCL washing solution, 250 µL of blocking buffer was added thereto, and the plate was incubated at room temperature for 1 hour. Next, the plate was washed three times with 300 µL of HISCL washing solution, 50 µL of the competitive inhibition solution was added thereto, and the plate was incubated at room temperature for 1 hour. Thereafter, the plate was washed three times with 300 µL of HISCL washing solution, 50 µL of Peroxidase-conjugated AffinipureGoat Anti-Mouse IgG (Jackson ImmunoResearch Laboratories Inc.) diluted 5000-fold with blocking buffer was added thereto, and the plate was incubated at room temperature for 1 hour. Finally, the plate was washed nine times with 300 µL of HISCL washing solution, 50 µL of SuperSignal ELISA Pico Chemiluminescent Substrate (Thermo Fisher Scientific Inc.) was added thereto, the plate was incubated at room temperature for 5 minutes, and the luminescence intensity was measured with a microplate reader. The intensity of competition was evaluated as absorption rate, and was calculated by dividing the difference in signal intensity, that is decreased by adding the competitive peptide, by the signal intensity when no competitive peptide was added.

First, measurement was performed using a mixed solution of four types of peptides in the order of sequence number, as a competitive peptide. As shown in Fig. 9, it was revealed that a high absorption rate was observed when a mixture of SEQ ID NOs: 53 to 56 was added.

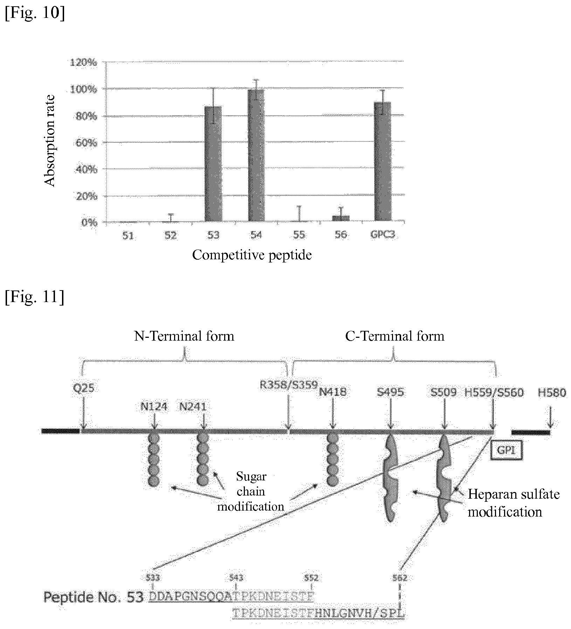

Next, measurement was performed independently using each peptide of the competitive peptide mixed solution in which inhibition was confirmed and each peptide of sequence numbers before and after those of the peptides of the competitive peptide mixed solution. As shown in Fig. 10, it was revealed that strong absorption rates were observed in SEQ ID NOs: 53 and 54.

Based on this, as shown in Fig. 11, a 543rd to 552nd sequence of GPC3, TPKDNEISTF, which is a sequence common to SEQ ID NOs: 53 and 54, was determined to be an epitope of a C-terminal recognition type monoclonal antibody.

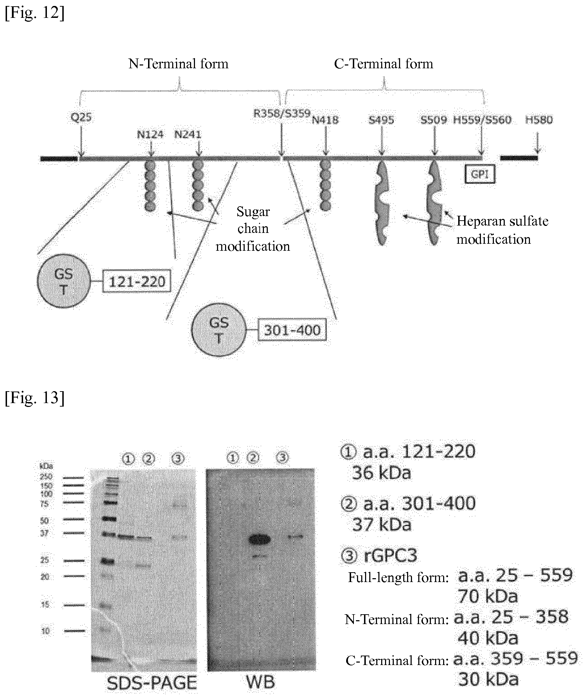

Epitope analysis of the N-terminal recognition type monoclonal antibody used for capture was performed by Western blot analysis using a partial recombinant protein as shown in Fig. 12.

5 ng each of full-length recombinant GPC3 and N-terminal partial recombinant GPC a.a. 121-220 (Abnova Corporation) and a.a. 301-400 (Abnova Corporation) were each taken, and each was dissolved in Sample Buffer Solution 2ME+ (Wako Pure Chemical Industries, Ltd) diluted 4-fold. This solution was heated at 98°C for 5 minutes, and then the whole amount to SuperSep Ace 15% 13 well (Wako Pure Chemical Industries, Ltd) was applied to perform electrophoresis. Thereafter, a gel was stained using SilverQuest Silver Staining Kit (Thermo Fisher Scientific Inc.). The operation was performed according to the instruction manual attached to the kit.

Also, a gel electrophoresed in the same manner was transferred to Immobilon-FL PVDF (Merck KGaA) at 4°C, 100 V for 1 hour. The membrane was immersed in Tris-buffered saline (TBS) containing 0.05 v/v% Tween-20, 40 mg/mL BSA, and shaken at room temperature for 1 hour. Further, the membrane was immersed in an N-terminal recognition type monoclonal antibody solution diluted to 0.5 µg/mL with Tris-buffered saline containing 0.1 v/v% Tween-20, 50 mg/mL skim milk, and shaken at room temperature for 1 hour.

Thereafter, the membrane was washed three times with Tris-buffered saline containing 0.05 v/v% Tween-20, immersed in a Polyclonal Goat Anti-Mouse Immunoglobulins/HRP solution diluted 1000-fold with Tris-buffered saline containing 0.1 v/v% Tween-20, 50 mg/mL skim milk, and shaken at room temperature for 1 hour.

Finally, the membrane was washed three times with Tris-buffered saline containing 0.05 v/v% Tween-20, and made luminescent using Pierce Western Blotting Substrate Plus (Thermo Fisher Scientific Inc.). The luminescent membrane was imaged using ImageQuant LAS4000mini (GE Healthcare). The operation was performed according to the instruction manual attached to the kit.

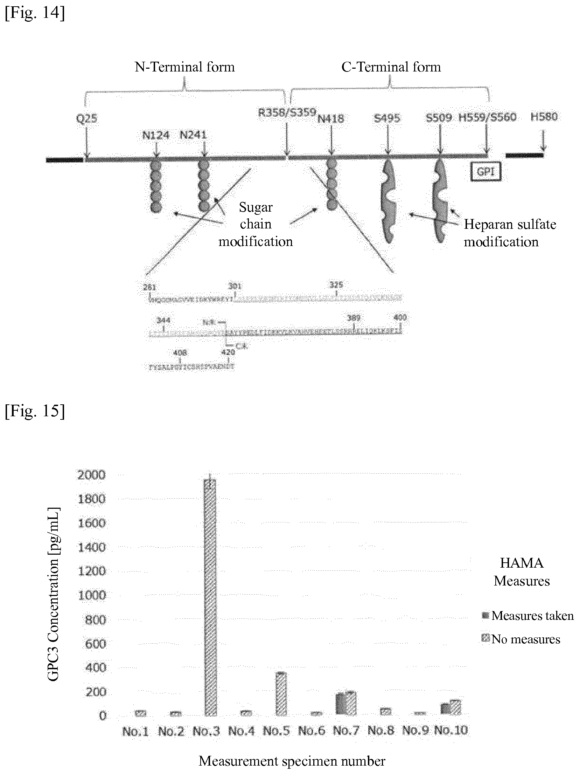

Fig. 13 shows the results of silver staining (left) and Western blot (right). Based on this result, it was found that the N-terminal recognition type monoclonal antibody used in this example reacts with 301-400 a.a. and 25-358 a.a of GPC3. Therefore, as shown in Fig. 14, 301-358 a.a that is a sequence common to both was determined to be an epitope region of the present antibody.

### Example 4: Verification of Effect of Addition of Non-Specific Reaction Inhibitor

Using a first N-terminal recognition type monoclonal antibody (biotin-labeled) that recognizes 301st to 358th peptides of GPC3 and a second C-terminal recognition type monoclonal antibody (alkaline phosphatase-labeled) that recognizes 543rd to 552nd peptides of GPC3, ten types of commercially available plasma (ProteoGenex) were measured with automated immunoassay apparatus HISCL-800 (Sysmex Corporation). Biotin labeling of the antibody was performed using Biotin Labeling Kit-SH (Dojindo Laboratories) according to the attached protocol.

As a sample for preparing a calibration curve, the same one as in ELISA was used. For dilution solutions of each antibody, those having dilution rates similar to those of ELISA were prepared, and there were prepared two types of dilution solvents, blocking buffer for ELISA (no measures for non-specificity) and blocking buffer (measures for non-specificity are taken) to which mouse IgG at the final concentration of 2 mg/mL was added.

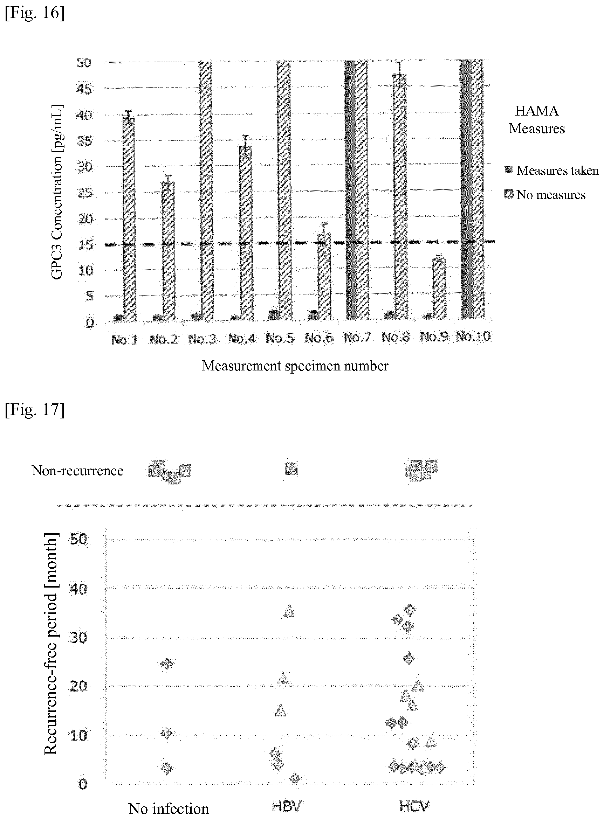

As shown in Fig. 15 (and Fig. 16 of its enlarged view), it can be seen that, when no non-specific reaction inhibitor was added, all specimens exceeded the threshold value of 15 pg/mL, and thus a false positive may occur. In Figs. 15 and 16, "HAMA" represents Human Anti-Mouse Antibody.

### Example 5: Prediction of Early Recurrence for Hepatitis B Patients

Among 39 HCC patient blood samples, 28 cases in which recurrence was observed within 4 years were analyzed based on the presence or absence of virus infection. The recurrence group had a median age of 68 years (63 to 72), of which 18 cases were HCV positive, 7 cases were HBV positive, and 3 cases had no virus infection. The median of the recurrence-free period was 10 months (3 to 21).

As specific experimental procedures, first, a commercially available GPC3 N-terminal recognition type monoclonal antibody was biotinylated using Biotin Labeling Kit-SH (Dojindo Laboratories). Next, a commercially available GPC3 C-terminal recognition type monoclonal antibody was conjugated with ALP using Alkaline phosphatase Labeling Kit-SH (Dojindo Laboratories). The biotinylated antibody obtained above, a measurement sample and a non-specific reaction inhibitor were suspended, and streptavidin-modified HISCL magnetic beads were added to the resulting suspension, and suspended. The beads were collected to remove supernatant, and the beads were washed with HISCL washing solution to perform B/F separation. The obtained beads, the ALP-conjugated antibody and the non-specific reaction inhibitor obtained above were suspended, the beads were collected, and B/F separation was performed in the same manner as above. HISCL substrate buffer and HISCL luminescent substrate were suspended in the obtained beads, and the luminescence intensity was measured using HISCL-800. The amounts of the biotinylated antibody and the ALP-conjugated antibody to be used were adjusted so that 15 pg/mL full-length recombinant GPC3 (R&D Systems, Inc.) could be measured at S/N of 2 or more.

The results of comparing the recurrence-free periods for each type of virus infection for the GPC3-positive group and negative group are shown in Fig. 17. In Fig. 17, the upper side of the broken line represents the patients of non-recurrence group, and the lower side of the broken line represents the patients of recurrence group. In addition, diamonds represent the GPC3-positive group, triangles represent the GPC3-negative recurrence group, and squares particularly represent the GPC3-negative non-recurrence group. As is clear from Fig. 17, in the HBV-positive patients, the GPC3-positive group was found to have a short period until recurrence (recurrence-free period) (average 3.7 months) as compared to the GPC3-negative group in which recurrence was observed. Recurrence was also observed in all HBV positive and GPC3-positive patients.

### REFERENCE SIGNS LIST

- 11: Recurrence risk prediction assisting apparatus
- 22: Measurement device
- 33: Determination device
- 33a: Computer main body
- 33b: Input unit
- 33c: Display unit
- 301: Receiving unit
- 302: Storage unit
- 303: Calculation unit
- 304: Determination unit
- 305: Output unit
- 330: CPU
- 331: ROM
- 332: RAM
- 333: Hard disk
- 334: Input/output interface
- 335: Reading device
- 336: Communication interface
- 337: Image output interface
- 338: Bus
- 340: Recording medium
- 500: Box
- 501: First reagent container
- 502: Second reagent container
- 503: Package insert

## Claims

1. A method for assisting prediction of recurrence risk for hepatocellular carcinoma patients, comprising steps of:
measuring concentration of glypican 3 (GPC3) peptide recognized by both a first monoclonal antibody and a second monoclonal antibody in a blood sample of a hepatocellular carcinoma patient, using the first monoclonal antibody that recognizes a peptide having an amino acid sequence in the N-terminal side of GPC3 and the second monoclonal antibody that recognizes a peptide having an amino acid sequence in the C-terminal side of GPC3, wherein the N- and C-terminal sides of GPC3 correspond respectively to the N- and C-terminal peptides obtained from cleavage of the full length GPC3 between the 358th and 359th amino acid; and
determining that recurrence risk of hepatocellular carcinoma is high when the concentration of GPC3 peptide is equal to or greater than a predetermined threshold value, and determining that recurrence risk of hepatocellular carcinoma is low when the concentration of GPC3 peptide is less than the predetermined threshold value.

2. The method according to claim 1, wherein the first monoclonal antibody is an antibody that recognizes a peptide having a sequence corresponding to the 301st to 358th amino acid of GPC3.

3. The method according to any one of claims 1 or 2, wherein the second monoclonal antibody is an antibody that recognizes a peptide having a sequence corresponding to the 543rd to 552nd amino acid of GPC3.

4. The method according to any one of claims 1 to 3, wherein the GPC3 peptide is a peptide containing the 301st to 552nd amino acid of GPC3.

5. The method according to any one of claims 1 to 4 wherein, in the determination step, among persons infected with hepatitis B virus having hepatocellular carcinoma, a GPC3-positive group is determined to have a high possibility of recurrence of hepatocellular carcinoma within a predetermined period.

6. The method according to any one of claims 1 to 5, wherein the measurement step is performed, in the presence of a non-specific reaction inhibitor.

7. Use of a kit comprising a first monoclonal antibody that recognizes a peptide having an amino acid sequence in the N-terminal side of GPC3 and a second monoclonal antibody that recognizes a peptide having an amino acid sequence in the C-terminal side of GPC3, wherein the N- and C-terminal side of GPC3 correspond to the N- and C-terminal peptides obtained from cleavage of the full length GPC3 between the 358th and 359th amino acid, for assisting prediction of recurrence risk for hepatocellular carcinoma patients in a method according to any one of claims 1 to 6.

## Patentansprüche

1. Verfahren zum Unterstützen der Vorhersage des Rezidivrisikos bei Patienten mit hepatozellulärem Karzinom, umfassend die folgenden Schritte:
Messen der Konzentration an Glypican 3(GPC3)-Peptid, das von sowohl einem ersten monoklonalen Antikörper als auch einem zweiten monoklonalen Antikörper erkannt wird, in einer Blutprobe eines Patienten mit hepatozellulärem Karzinom unter Verwendung des ersten monoklonalen Antikörpers, der ein Peptid mit einer Aminosäuresequenz in der N-terminalen Seite von GPC3 erkennt, und des zweiten monoklonalen Antikörpers, der ein Peptid mit einer Aminosäuresequenz in der C-terminalen Seite von GPC3 erkennt, wobei die N- und die C-terminale Seite von GPC3 jeweils dem N- und dem C-terminalen Peptid entsprechen, das aus Spaltung des GPC3 mit voller Länge zwischen der 358. und der 359. Aminosäure erhalten wird; und
Bestimmen, dass das Rezidivrisiko von hepatozellulärem Karzinom hoch ist, wenn die Konzentration an GPC3-Peptid gleich oder größer als ein vorab festgelegter Schwellenwert ist, und Bestimmen, dass das Rezidivrisiko von hepatozellulärem Karzinom gering ist, wenn die Konzentration an GPC3-Peptid niedriger als der vorab festgelegte Schwellenwert ist.

2. Verfahren nach Anspruch 1, wobei es sich bei dem ersten monoklonalen Antikörper um einen Antikörper handelt, der ein Peptid mit einer Sequenz entsprechend der 301. bis 358. Aminosäure von GPC3 erkennt.

3. Verfahren nach einem der Ansprüche 1 oder 2, wobei es sich bei dem zweiten monoklonalen Antikörper um einen Antikörper handelt, der ein Peptid mit einer Sequenz entsprechend der 543. bis 552. Aminosäure von GPC3 erkennt.

4. Verfahren nach einem der Ansprüche 1 bis 3, wobei es sich bei dem GPC3-Peptid um ein Peptid handelt, das die 301. bis 552. Aminosäure von GPC3 enthält.

5. Verfahren nach einem der Ansprüche 1 bis 4, wobei im Bestimmungsschritt unter mit Hepatitis-B-Virus infizierten Personen mit hepatozellulärem Karzinom bestimmt wird, dass bei einer GPC3-positiven Gruppe eine hohe Wahrscheinlichkeit eines Rezidivs des hepatozellulären Karzinoms innerhalb eines vorab festgelegten Zeitraums besteht.

6. Verfahren nach einem der Ansprüche 1 bis 5, wobei der Messschritt in Gegenwart eines nichtspezifischen Reaktionshemmers durchgeführt wird.

7. Verwendung eines Kits, das einen ersten monoklonalen Antikörper, der ein Peptid mit einer Aminosäuresequenz in der N-terminalen Seite von GPC3 erkennt, und einen zweiten monoklonalen Antikörper, der ein Peptid mit einer Aminosäuresequenz in der C-terminalen Seite von GPC3 erkennt, umfasst, wobei die N- und die C-terminale Seite von GPC3 dem N- und dem C-terminalen Peptid entsprechen, das aus Spaltung des GPC3 mit voller Länge zwischen der 358. und der 359. Aminosäure erhalten wird, zur Unterstützung der Vorhersage des Rezidivrisikos bei Patienten mit hepatozellulärem Karzinom in einem Verfahren nach einem der Ansprüche 1 bis 6.

## Revendications

1. Procédé destiné à assister la prédiction d'un risque de récurrence pour des patients atteints d'un carcinome hépatocellulaire, comprenant les étapes consistant à :
mesurer la concentration d'un peptide de glypican 3 (GPC3) reconnu à la fois par un premier anticorps monoclonal et un deuxième anticorps monoclonal dans un échantillon sanguin d'un patient atteint d'un carcinome hépatocellulaire, en utilisant le premier anticorps monoclonal, qui reconnaît un peptide ayant une séquence d'acides aminés dans le côté N-terminal du GPC3, et le deuxième anticorps monoclonal qui reconnaît un peptide ayant une séquence d'acides aminés dans le côté C-terminal du GPC3, dans lequel les côtés N- et C-terminaux du GPC3 correspondent respectivement aux peptides N- et C-terminaux obtenus à partir d'une coupure du GPC3 de longueur totale entre le 358^{ème} et le 359^{ème} acides aminés ; et
déterminer que le risque de récurrence d'un carcinome hépatocellulaire est élevé quand la concentration en peptide de GPC3 est supérieure ou égale à une valeur seuil prédéterminée et déterminer que le risque de récurrence d'un carcinome hépatocellulaire est faible quand la concentration en peptide de GPC3 est inférieure à la valeur seuil prédéterminée.

2. Procédé selon la revendication 1, dans lequel le premier anticorps monoclonal est un anticorps qui reconnaît un peptide ayant une séquence correspondant au 301^{ème} jusqu'au 358^{ème} acides aminés du GPC3.

3. Procédé selon l'une quelconque des revendications 1 ou 2, dans lequel le deuxième anticorps monoclonal est un anticorps qui reconnaît un peptide ayant une séquence correspondant au 543^{ème} jusqu'au 552^{ème} acides aminés du GPC3.

4. Procédé selon l'une quelconque des revendications 1 à 3, dans lequel le peptide de GPC3 est un peptide contenant du 301^{ème} jusqu'au 552^{ème} acides aminés du GPC3.

5. Procédé selon l'une quelconque des revendications 1 à 4, dans lequel, dans l'étape de détermination, parmi des personnes infectées par un virus de l'hépatite B étant atteintes d'un carcinome hépatocellulaire, on détermine qu'un groupe positif pour le GPC3 a une possibilité élevée de récurrence d'un carcinome hépatocellulaire dans une période prédéterminée.

6. Procédé selon l'une quelconque des revendications 1 à 5, dans lequel l'étape de mesure est exécutée en présence d'un inhibiteur réactionnel non spécifique.

7. Utilisation d'une trousse comprenant un premier anticorps monoclonal, qui reconnaît un peptide ayant une séquence d'acides aminés dans le côté N-terminal du GPC3, et un deuxième anticorps monoclonal qui reconnaît un peptide ayant une séquence d'acides aminés dans le côté C-terminal du GPC3, dans laquelle les côtés N- et C-terminaux du GPC3 correspondent aux peptides N- et C-terminaux obtenus à partir d'une coupure du GPC3 de longueur totale entre le 358^{ème} et le 359^{ème} acides aminés, pour assister la prédiction d'un risque de récurrence pour des patients atteints d'un carcinome hépatocellulaire dans un procédé selon l'une quelconque des revendications 1 à 6.
